# EUROPEAN PATENT APPLICATION

(11) **EP 4 306 522 A1**
(43) Date of publication of application: **17.01.2024**
(21) Application number: 22766398.6
(22) Date of filing: 11.03.2022
(51) Int. Cl.: C07D 417/04, A61K 31/437, A61P 25/28

(54) **FUSED RING HETEROCYCLIC COMPOUND AND APPLICATION THEREOF, AND PHARMACEUTICAL COMPOSITION CONTAINING SAME AND APPLICATION THEREOF**

(30) Priority: 11.03.2021 CN 202110267929; 11.03.2021 CN 202110267927; 11.03.2021 CN 202110267914
(71) Applicant: Zhejiang University, Hangzhou City, Zhejiang 310058 (CN)
(72) Inventor: YANG, Wei, Hangzhou city, Zhejiang 310058 (CN); ZOU, Hongbin, Hangzhou city, Zhejiang 310058 (CN); YU, Peilin, Hangzhou city, Zhejiang 310058 (CN); LI, Jinbiao, Hangzhou city, Zhejiang 310058 (CN); ZHANG, Yi, Hangzhou city, Zhejiang 310058 (CN); FANG, Qiuyuan, Hangzhou city, Zhejiang 310058 (CN); YE, Peiwu, Hangzhou city, Zhejiang 310058 (CN)
(74) Representative: Viering, Jentschura & Partner mbB Patent- und Rechtsanwälte
(86) International application number: PCT/CN2022/080442
(87) International publication number: WO 2022/188876

(57) **Abstract**

The present application discloses a fused heterocyclic compound and application thereof, as well as a pharmaceutical composition containing same and application thereof. In the present application, the fused heterocyclic compound, or its pharmaceutically acceptable salt, stereoisomer, solvent compound, or prodrug, has the structure shown in general formula (I) and can be used as a TRPM2 inhibitor for the preparation of drugs for treating TRPM2 dysfunctional diseases.

## Description

### Cross-reference to related applications

This patent application claims priority for the Chinese patent application filed on March 11, 2021, with application number 2021102679290 and named "Fused heterocyclic compound and application thereof, pharmaceutical composition containing same and application thereof". The contents of the above application are incorporated by reference herein.

This patent application claims priority for the Chinese patent application filed on March 11, 2021, with application number 2021102679271 and named " Application of a fused heterocyclic compound or a pharmaceutically acceptable salt thereof in the preparation of cerebral stroke drugs". The contents of the above application are incorporated by reference herein.

This patent application claims priority for the Chinese patent application filed on March 11, 2021, with application number 2021102679144 and named " Application of a fused heterocyclic compound or pharmaceutically acceptable salt thereof in the preparation of drugs for treating Parkinson's disease". The contents of the above application are incorporated by reference herein.

### Technical field

The embodiments of the present invention relate to the field of pharmaceutical chemistry, in particular to a fused heterocyclic compound and application thereof, pharmaceutical composition containing same and application thereof.

### Background technique

TRPM2 (Transient Receptor Potential Melastatin 2) is a permeable homotetramer non-selective cation channel in the family of transient receptor potential ion channels, which is widely expressed in cells in various parts of the human body, such as: brain, heart, blood vessels, kidney, endothelial cells, smooth muscle cells and immune cells. Extracellular signals such as oxidative stress, tumor necrosis factor-α (TNF-α), amyloid β-peptide (Aβ), and concomitant cutinoglobulin A (ConA) can lead to the production of intracellular adenosine diphosphate ribose (ADPR), which, as a proven endogenous ligand for TRPM2, the increased level of ADPR will activate TRPM2 channels, causing the flow of cations such as Na⁺, K⁺, Zn²⁺ and Ca²⁺ into the cell, which will have an impact on the ion homeostasis in the cell.

In recent years, in-depth studies on the TRPM2 channel function have found that it mediates a number of important physiological and pathological functions: 1) participating in the defervescence regulation of fever and regulating the adaptability of mice to external temperature (Song K et al., Science, 2016; Tan CH et al., Nature, 2016); 2) mediating a variety of important immune and inflammatory responses, for example, mediating anti-swollen lung inflammation triggered by anti-tumor drugs (Yonezawa R et al., Free Radic Biol Med, 2016); 3) regulating the internal flow of calcium-ion, cytokine and chemokine release, and reactive oxygen species (ROS) levels, and mediating the genesis and development of neurological diseases such as Parkinson's disease, Alzheimer's disease, amyotrophic lateral sclerosis, spinal cord injury and pain,etc (Hermosura MC et al, Proc Natl Acad Sci USA, 2008; Naziroǧlu M et al, J Recept Signal Transduct Res, 2012; Ostapchenko VG et al, JNeurosci, 2015); 4) participating in the regulation of insulin secretion and blood glucose levels, mediating the genesis of diabetes mellitus (Uchida K et al, Diabetes, 2011); 5) functioning in the genesis and development of a variety of malignant tumors such as breast, bladder, neuroblastoma, and prostate cancers through the regulation of its own expression level (Hopkins MM et al, Int J Oncol, 2015; Cao QF et al, Cancer Biother Radiopharm, 2015; Chen SJ et al, Am J Physiol Cell Physiol, 2013; Zeng X et al, Prostate Cancer Prostatic Dis, 2010).

In summary, the participating in the regulation of various pathophysiological functions of TRPM2 channel is a potential therapeutic target for a variety of diseases, and studies have shown that a variety of diseases involving the channel are caused by over-activation of the channel. Therefore, the development of inhibitors of these channels is of great importance for the further elucidation of the channel function, the confirmation of its therapeutic targets for a variety of diseases, and the research and development of drugs for this target.

### SUMMARY OF THE INVENTION

As used herein, the term 'pharmaceutically acceptable salt' refers to a salt of a compound of the present invention that is pharmaceutically acceptable and has pharmacological activity of the parent compound. This type of salt includes salts added to inorganic acids or acids formed with organic acids, the organic acids are such as nitric acid, phosphoric acid, carbonic acid, etc; The organic acids are such as propionic acid, caproic acid, cyclopentanoic acid, glycolic acid, pyruvic acid, gluconic acid, stearic acid, muconic acid, etc; or a salt formed when acidic protons present on the parent compound are replaced by metal ions, such as alkali metal ions or alkaline earth metal ions; or coordination compounds formed with organic bases, such as ethanolamine, diethanolamine, triethanolamine, N-methylglucosamine, etc. The pharmaceutically acceptable salts of the present invention can be synthesized by conventional chemical methods from parent compounds containing acid or base groups. In general, the preparation method of such salts is that the salts are prepared by reacting these compounds in the form of free acids or bases with stoichiometric appropriate bases or acids in water, organic solvents, or a mixture of both. Generally, non aqueous media such as ether, ethyl acetate, ethanol, isopropanol, or acetonitrile are preferred. In addition to the form of salt, the compounds provided by the present invention also exist in the form of prodrugs. The prodrug of the compound described herein easily undergoes chemical changes under physiological conditions, thereby transforming into the compound of the invention. In addition, precursor drugs can be converted into the compounds of the present invention through chemical or biochemical methods in the in vivo environment.

As used in this article, the term "stereoisomer" includes conformational isomers and configurational isomers, wherein the configurational isomers mainly include cis-trans isomers and optical isomers. The compound described in the present invention can exist in the form of stereoisomers and therefore covers all possible forms of stereoisomers, including but not limited to cis-trans isomer, tautomer, enantiomer, diastereoisomer, atropisomer, etc. The compound described in the present invention can also exist in any combination or mixture of the aforementioned stereoisomer, for example in the form of an equal mixture of mesomer, racemes and atropisomer. For example, the compound is a single enantiomer, a single diastereoisomer or a mixture thereof, or a single atropisomer or a mixture thereof.

As used in this article, the term "solvent compound" refers to a substance formed by the combination of a compound of the present invention with a pharmaceutically acceptable solvent. The pharmaceutically acceptable solvent includes water, ethanol, acetic acid, etc. Solvent compounds include both chemically calculated and non chemically calculated solvent compounds, preferably hydrates. Some compounds of the present invention can exist in non-solvated or solvated forms, including hydrate forms. Generally speaking, the solvated form is equivalent to the non-solvated form and is included within the scope of the present invention.

As used herein, the terms "heteroaryl group", "heteroaryl ring", and "heteroaromatic ring" are interchangeable, referring to a single or fused multi ring (i.e., a shared adjacent ring atomic pair, which can be C-C or N-C) group where the ring atom is substituted by at least one heteroatom independently selected from nitrogen, oxygen, or sulfur, wherein the nitrogen and sulfur atoms can be optionally oxidized and the nitrogen atoms can be optionally quaternized. The term " pentamembered heteroaryl group " used herein refers to a heteroaryl group with five ring atoms, and the term " hexamembered heteroaryl group " refers to a heteroaryl group with six ring atoms. The terms " pentamembered heteroaryl group " and " hexamembered heteroaryl group " used herein are not limited to the following forms:

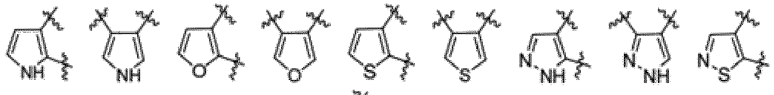

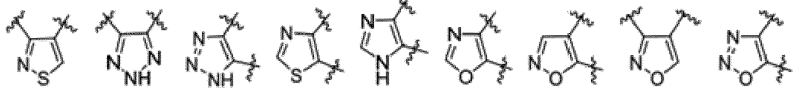

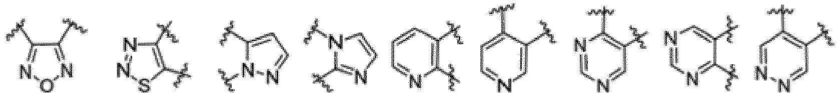

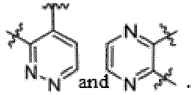

As used herein, the term "heterocyclic group" refers to a ring group where at least one carbon atom in a single ring is substituted by a heteroatom, which is a non carbon atom, preferably an N, O, or S atom.

The purpose of an embodiment of the present invention is to provide a fused heterocyclic compound, which can be used as a TRPM2 inhibitor and apply to prepare a medicine for treating TRPM2 dysfunctional diseases.

To solve the above technical problem, a first aspect of the present invention provides a fused heterocyclic compound or a pharmaceutically acceptable salt, stereoisomer, solvate or prodrug thereof, wherein the fused heterocyclic compound has the structure shown in a general formula (I): 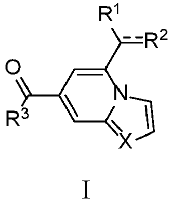
in the formula (1), R¹ is a heteroaryl group unsubstituted or with at least one hydrogen atom substituted by R¹⁻¹, or R¹ is a phenyl group unsubstituted or with at least one hydrogen atom substituted by R¹⁻², wherein R¹⁻¹ is a halogen or C₁₋₆ alkyl group, and R¹⁻² is a halogen, cyano, nitro group, C₂₋₈ ester group, C₁₋₆ alkyl group, C₁₋₆ alkoxy group;
represents a single or double bond, and when -- is a single bond, R² is a hydroxyl group, C₂₋₁₂ acyloxy group, or C₁₋₆ alkoxy group; when -- is a double bond, R² is an oxygen atom;
R³ is a hydroxyl group, C₁₋₄ alkyl group, C₁₋₆ alkoxy group, a heterocyclic group Ra containing at least one N, O, and/or S atom, or 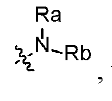 , wherein Ra and Rb are independently selected from hydrogen, C₁₋₄ alkyl group, or C₁₋₄ alkoxy group, respectively;
X is a carbon atom or a nitrogen atom.

Based on obtaining better technical effects, the fused heterocyclic compound described in the present invention does not include the following structures:

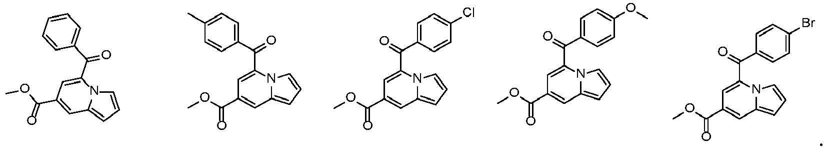

In some preferred embodiments, the R¹ is a heteroaryl group unsubstituted or with at least one hydrogen atom substituted by R¹⁻¹, which is a pentamembered heteroaryl group unsubstituted or with at least one hydrogen atom substituted by R¹⁻¹, or a hexamembered heteroaryl group unsubstituted or with at least one hydrogen atom substituted by R¹⁻¹; wherein the R¹⁻¹ is a halogen or C₁₋₆ alkyl group, the halogen is preferably fluorine, chlorine, bromine, or iodine, the C1-6 alkyl group is preferably C₁₋₄ alkyl group(such as: methyl, ethyl, n-propyl, isopropyl, n-butyl, isobutyl or tert-butyl);
The pentamembered heteroaryl group unsubstituted or with at least one hydrogen atom substituted by R¹⁻¹ is preferably a pentamembered heterocyclic group unsubstituted or with at least one hydrogen atom ubstituted by R¹⁻¹ containing at least one N, O, and/or S atom; and more preferably a pentamembered heterocyclic group unsubstituted or with at least one hydrogen atom substituted by R¹⁻¹ containing at least one O, and/or S atom; most preferably a furanyl or thiophenyl group, the furanyl group is preferably 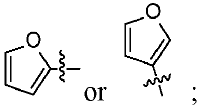 the thiophenyl group is preferably 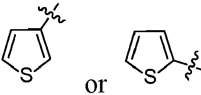 ; (the number of the R¹⁻¹ can be one or more, and when there are multiple R¹⁻¹, the R¹⁻¹ is same or different).

Alternatively, the R¹ is a phenyl group unsubstituted or with at least one hydrogen atom has been replaced by R1-2 (the number of the R¹⁻² can be one or more, and when there are multiple R1-2, the R¹⁻² is same or different), the R¹⁻² is a halogen, cyano, nitro group, C₁₋₆ alkyl group, or C₁₋₆ alkoxy group; the halogen is preferably fluorine, chlorine, bromine, or iodine; the C₁₋₆ alkyl is preferably C₁₋₄ alkyl (such as methyl, ethyl, n-propyl, isopropyl, n-butyl, isobutyl, or tert butyl); the C₁₋₆ alkoxy group is preferably a C₁₋₄ alkoxy group (such as methoxy or ethoxy).

In some preferred embodiments, -- represents a single bond, R² is a hydroxyl group, C₂₋₁₂ acyloxy group, or C₁₋₆ alkoxy group; the C₂₋₁₂ acyloxy group is preferably C₂₋₈ acyloxy group, the C₂₋₈ acyloxy group is 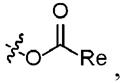 wherein the Re is C₁₋₇ alkyl(such as methyl, ethyl, propyl, butyl, pentyl, hexyl or heptyl); the C₁₋₆ alkoxy group is preferably C₁₋₄ alkoxy group (such as methoxy, ethoxy or propoxy);
alternatively, -- represents a double bond, R² is an oxygen atom.

In some preferred embodiments, R³ is a hydroxyl group, C₁₋₄ alkyl group, C₁₋₆ alkoxy group, 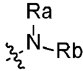 or a heterocyclic group containing at least one N, O, and/or S atom, wherein Ra and Rb are independently selected from hydrogen, C₁₋₄ alkyl group, or C₁₋₄ alkoxy group respectively; the C₁₋₄ alkyl group is preferably methyl, ethyl, n-propyl, isopropyl, n-butyl, isobutyl, or tert butyl; the C₁₋₆ alkoxy group is preferably C₁₋₄ alkoxy group (such as methoxy , ethoxy or propoxy); in the 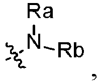 Ra and Rb are respectively independently preferably hydrogen, methyl, ethyl, methoxy or ethoxy(such as 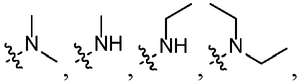 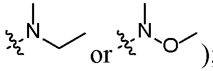 ; the heterocyclic group containing at least one N, O, and/or S atom is preferably a ternary heterocyclic group containing at least one N, O, and/or S atom, a quaternary heterocyclic group containing at least one N, O, and/or S atom, a pentamembered heterocyclic group containing at least one N, O, and/or S atom, or a hexamembered heterocyclic group containing at least one N, O, and/or S atom, more preferably a quaternary heterocyclic group containing at least one N and/or O atom, a pentamembered heterocyclic group containing at least one N and/or O atom, or a hexamembered heterocyclic group containing at least one N and/or O atom, further preferably a quaternary heterocyclic group containing one or two N and/or O atoms,
a pentamembered heterocyclic group containing one or two N and/or O atoms, a hexamembered heterocyclic group containing one or two N and/or O atoms, most preferably a four to six membered cyclic imino group(such as 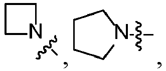 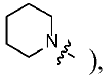 a hexamembered heterocyclic group containing two N atoms, preferably 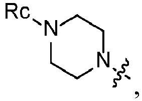 wherein Rc is selected from hydrogen or C₁₋₄ alkoxy group(such as 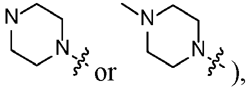 or a hexamembered heterocyclic group containing one N atom and one O atom(such as

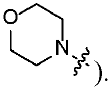

In some preferred embodiments, X is a carbon atom, the fused heterocyclic compound has a structure shown in a general formula (II): 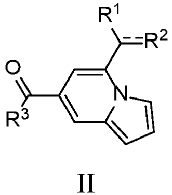
in the formula(II), R¹ is a heteroaryl group unsubstituted or with at least one hydrogen atom substituted by R¹⁻¹, or R¹ is a phenyl group unsubstituted or with at least one hydrogen atom substituted by R¹⁻², wherein R¹⁻¹ is a halogen or C₁₋₆ alkyl group, the C₁₋₆ alkyl group
and R¹⁻² is a halogen, cyano, nitro group, C₁₋₆ alkyl group, the C₁₋₆ alkoxy group is preferably a C₁₋₄ alkyl group (such as methyl, ethyl, n-propyl, isopropyl, n-butyl, isobutyl, or tert butyl); the C₁₋₆ alkoxy group is preferably C₁₋₄ alkoxy group (such as methoxy or ethoxy);
represents a single or double bond, and when -- is a single bond, R² is a hydroxyl group, C₂₋₁₂ acyloxy group, or C₁₋₆ alkoxy group; When -- is a double bond, R² is an oxygen atom; the C₂₋₁₂ acyloxy group is preferably C₂₋₈ acyloxy group, the C₂₋₈ acyloxy group is 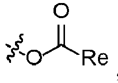 wherein Re is C₁₋₇ alkyl group(such as methyl, ethyl, propyl, butyl, pentyl, hexyl, or heptyl); the C₁₋₆ alkoxy group is C₁₋₄ alkoxy group(such as methoxy, ethoxy or propoxy);
R³ is a hydroxyl group, C₁₋₄ alkyl group, C₁₋₆ alkoxy group, 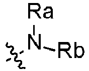 or a heterocyclic group containing at least one N, O and/or S atom, wherein Ra and Rb are independently selected from hydrogen, C₁₋₄ alkyl group, or C₁₋₆ alkoxy group respectively; the C₁₋₄ alkyl group is preferably methyl, ethyl, n-propyl, isopropyl, n-butyl, isobutyl, or tert butyl; the C₁₋₆ alkoxy group is preferably a C₁₋₄ alkoxy group (such as methoxy, ethoxy or propoxy); in the 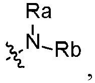 Ra and Rb are respectively independently preferably hydrogen, methyl, ethyl, methoxy or ethoxy(such as 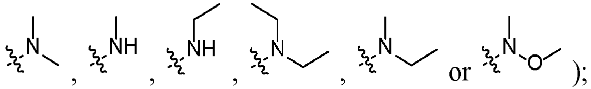 the heterocyclic group containing at least one N, O, and/or S atom is preferably a ternary heterocyclic group containing at least one N, O, and/or S atom, a quaternary heterocyclic group containing at least one N, O, and/or S atom, a pentamembered heterocyclic group containing at least one N, O, and/or S atom, or a hexamembered heterocyclic group containing at least one N, O, and/or S atom, more preferably a quaternary heterocyclic group containing at least one N and/or O atom, a pentamembered heterocyclic group containing at least one N and/or O atom, or a hexamembered heterocyclic group containing at least one N and/or O atom, further preferably a quaternary heterocyclic group containing one or two N and/or O atoms, a pentamembered heterocyclic group containing one or two N and/or O atoms, a hexamembered heterocyclic group containing one or two N and/or O atoms, most preferably a four to six membered cyclic imino group(such as 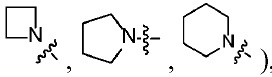 , a hexamembered heterocyclic group containing two N atoms, preferably 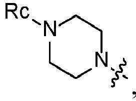 , wherein Rc is selected from hydrogen or C₁₋₄ alkoxy group(such as 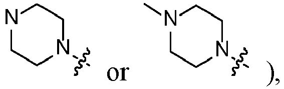 or a hexamembered heterocyclic group containing one N atom and one O atom(such

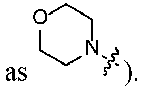

In some preferred embodiments, X is a carbon atom, -- is a single bond, the fused heterocyclic compound has a structure shown in a general formula (III): 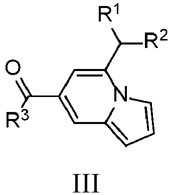
in the formula (III), R¹ is a phenyl group unsubstituted or with at least one hydrogen atom substituted by R¹⁻², wherein R¹⁻² is a halogen, cyano, nitro group, C₁₋₆ alkyl group, C₁₋₆ alkoxy group; preferably, R¹ is a phenyl unsubstituted;
R² is a hydroxyl group, C₂₋₁₂ acyloxy group, or C₁₋₆ alkoxy group; preferably, R² is a hydroxyl group, C₂₋₈ acyloxy group, or C₁₋₄ alkoxy group; the C₂₋₈ acyloxy group is 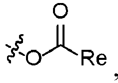 wherein Re is C₁₋₇ alkyl group (such as methyl, ethyl, propyl, butyl, pentyl, hexyl, or heptyl); the C₁₋₄ alkoxy group is preferably methoxy, ethoxy or propoxy;
R³ is a hydroxyl group, C₁₋₄ alkyl group, C₁₋₆ alkoxy group, 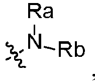 wherein Ra and Rb are independently selected from hydrogen, C₁₋₄ alkyl group, or C₁₋₄ alkoxy group respectively; preferably, R³ is a hydroxyl group, C₁₋₄ alkoxy group, 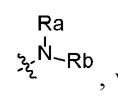 wherein Ra and Rb are independently selected from hydrogen or C₁₋₄ alkyl group respectively; more preferably R³ is a hydroxyl group, methoxy, ethoxy,

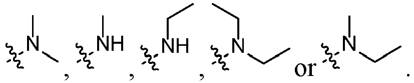

In some preferred embodiments, X is a carbon atom, -- is a double bond, the fused heterocyclic compound has a structure shown in a general formula (IV): 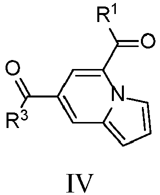
in the formula (IV), R¹ is a heteroaryl group unsubstituted or with at least one hydrogen atom substituted by R¹⁻¹, or R¹ is a phenyl group unsubstituted or substituted by R¹⁻², wherein R¹⁻¹ is a halogen or C₁₋₆ alkyl group, and R¹⁻² is a halogen, cyano, nitro group, C₁₋₆ alkyl group, C₁₋₆ alkoxy group;
R³ is a hydroxyl group, C₁₋₄ alkyl group, C₁₋₆ alkoxy group, a heterocyclic group containing at least one N, O, and/or S atom, or 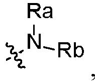 wherein Ra and Rb are independently selected from hydrogen, C₁₋₄ alkyl group, or C₁₋₄ alkoxy group respectively; preferably, R³ is a hydroxyl group, C₁₋₆ alkoxy group, a quaternary heterocyclic group containing at least one N and/or O atom, a pentamembered heterocyclic group containing at least one N and/or O atom, or a hexamembered heterocyclic group containing at least one N and/or O atom, or 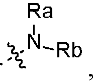 wherein Ra and Rb are independently selected from hydrogen, methyl, ethyl, methoxy or ethoxy respectively; more preferably, R³ is a hydroxyl group, methoxy, ethoxy, propoxy, four to six membered cyclic imino group(such as 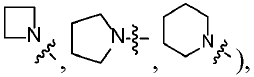 a hexamembered heterocyclic group containing two N atoms(such as 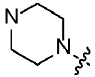 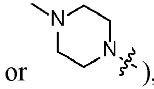 , or a hexamembered heterocyclic group containing one N atom and one O atom(such as

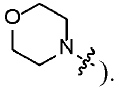

In some preferred embodiments, X is a carbon atom, -- is a double bond, the fused heterocyclic compound has a structure shown in a general formula (IV): 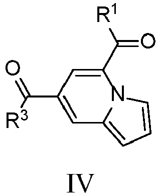
in the formula (IV), R¹ is a pentamembered heterocyclic group unsubstituted, a hexamembered heterocyclic group,unsubstituted, a phenyl group unsubstituted or with at least one hydrogen atom substituted by R¹⁻², wherein R¹⁻² is a halogen, C₁₋₆ alkyl group, C₁₋₆ alkoxy group; the halogen is preferably fluorine, chlorine, or bromine; the C₁₋₆ alkyl group is preferably a C₁₋₄ alkyl group(such as methyl, ethyl, n-propyl, isopropyl, n-butyl, isobutyl, or tert butyl); the C₁₋₆ alkoxy group is preferably a C₁₋₄ alkoxy group (such as methoxy);
R³ is a C₁₋₄ alkyl group, or C₁₋₆ alkoxy group; wherein the C₁₋₄ alkyl group is preferably methyl, ethyl, n-propyl, isopropyl, n-butyl, isobutyl, or tert butyl (for example methyl); the C₁₋₆ alkoxy group is preferably C₁₋₄ alkoxy group (such as methoxy, ethoxy or propoxy).

In some preferred embodiments, X is a carbon atom, -- is a double bond, the fused heterocyclic compound has the structure shown in a general formula (IV): 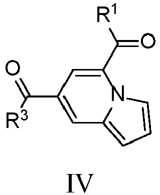
in the formula (IV), R¹ is a hexamembered heteroaryl group unsubstituted, or a phenyl group unsubstituted or with at least one hydrogen atom substituted by R¹⁻², wherein R¹⁻² is a halogen(such as fluorine);
R³ is a hydroxyl group.

In some preferred embodiments, X is a carbon atom, -- is a double bond, the fused heterocyclic compound has the structure shown in a general formula (IV): 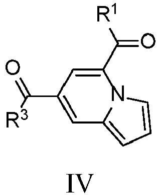
in the formula (IV), R¹ is a hexamembered heteroaryl group unsubstituted, or a phenyl group unsubstituted or with at least one hydrogen atom substituted by R¹⁻², wherein R¹⁻² is a halogen(such as fluorine);
R¹ is 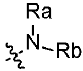 or a heterocyclic group containing at least one N , O and/or S atom, wherein Ra and Rb are respectively independently preferably hydrogen, methyl, ethyl, methoxy or ethoxy(such as 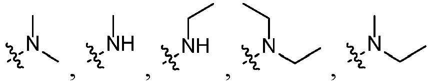 or 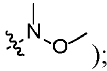 the heterocyclic group containing at least one N , O and/or S atom is preferably four to six membered cyclic imino group(such as 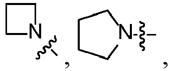 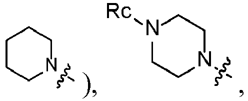 wherein Rc is selected from hydrogen or C₁₋₄ alkoxy group(such as 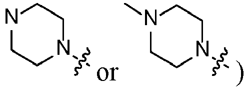 or a hexamembered heterocyclic group containing one N atom and one O atom(such as

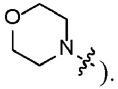

In some preferred embodiments, X is a carbon atom, -- is a double bond, the fused heterocyclic compound has the structure shown in a general formula (IV): 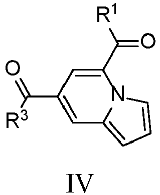 in the formula (IV), the R¹ is 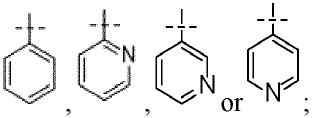 R³ is

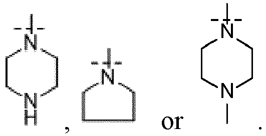

In some preferred embodiments, the fused heterocyclic compound is selected from any of the following structures:

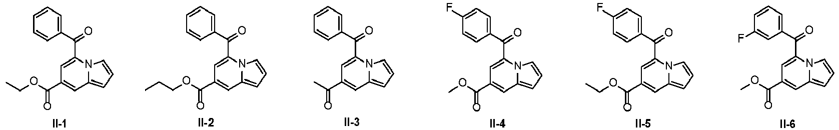

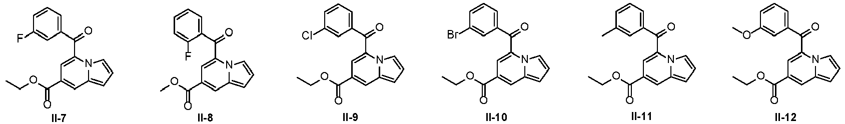

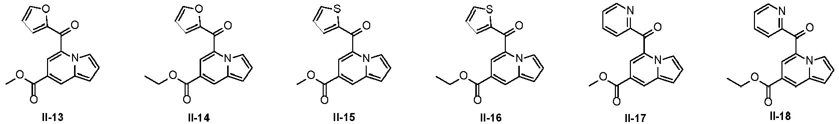

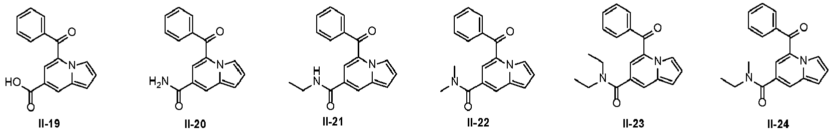

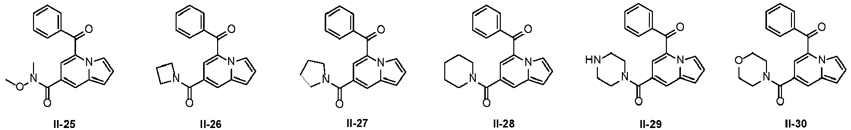

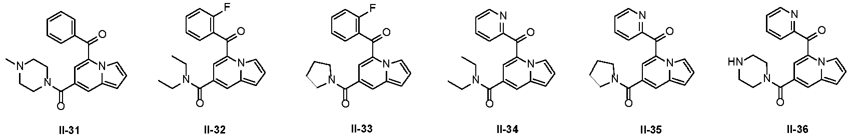

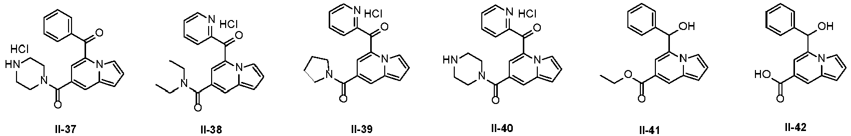

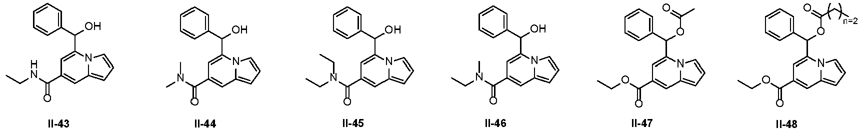

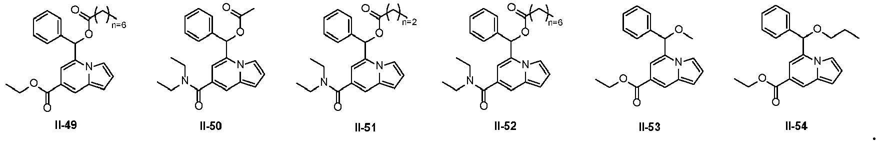

In some preferred embodiments, X is an N atom, -- is a single bond, the fused heterocyclic compound has the structure shown in a general formula (V): 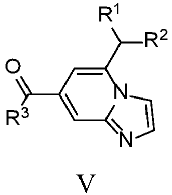
in the formula (V), R¹ is a phenyl group unsubstituted or with at least one hydrogen atom substituted by R¹⁻², wherein R¹⁻² is a halogen, cyano, nitro group, C₁₋₆ alkyl group, or C₁₋₆ alkoxy group; preferably, R¹ is a phenyl group unsubstituted or with at least one hydrogen atom substituted by halogen or C₁₋₄ alkyl, more preferably, R¹ is a phenyl group unsubstituted;
R² is a hydroxyl group or C₁₋₆ alkoxy group; preferably hydroxyl;
R³ is a hydroxyl group, C₁₋₄ alkyl group or C₁₋₆ alkoxy group; preferably C₁₋₆ alkoxy group; more preferably C₁₋₄ alkoxy (such as ethoxy).

In some preferred embodiments, X is an N atom, -- is a double bond, the fused heterocyclic compound has the structure shown in a general formula (VI): 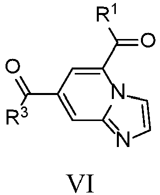
in the formula (VI), R¹ is a phenyl group unsubstituted or with at least one hydrogen atom substituted by R¹⁻², wherein R¹⁻² is a halogen, cyano, nitro group, C₁₋₆ alkyl group or C₁₋₆ alkoxy group; preferably, R¹ is phenyl group unsubstituted or with at least one hydrogen atom substituted by a halogen or C₁₋₄ alkyl group;
R³ is a hydroxyl group, C₁₋₄ alkyl group, C₁₋₆ alkoxy group, a heterocyclic group containing at least one N atom, or 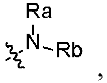 wherein Ra and Rb are independently selected from hydrogen or C₁₋₄ alkyl group respectively; preferably, R³ is hydroxyl group, C₁₋₄ alkyl group(such as methyl, ethyl, n-propyl, isopropyl, n-butyl, isobutyl, or tert butyl), C₁₋₄ alkoxy group (such as ethoxy), five to six membered cyclic imino group(such as 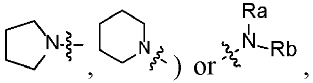 wherein Ra and Rb are independently selected from hydrogen methyl or ethyl 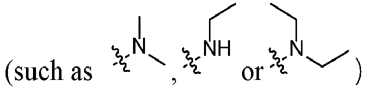 respectively.

In some preferred embodiments, X is an N atom, -- is a double bond, the fused heterocyclic compound has the structure shown in a general formula (VI): 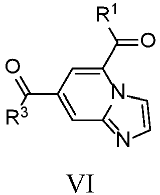
in the formula (VI), R¹ is a phenyl group unsubstituted or with at least one hydrogen atom substituted by halogen;
R³ is a hydroxyl group, C₁₋₄ alkyl group(such as methyl, ethyl, n-propyl, isopropyl, n-butyl, isobutyl, or tert butyl), or C₁₋₄ alkoxy group (such as ethoxy).

In some preferred embodiments, the fused heterocyclic compound is selected from any of the following structures:

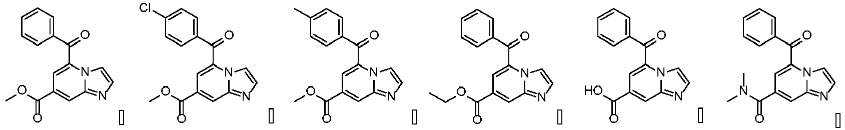

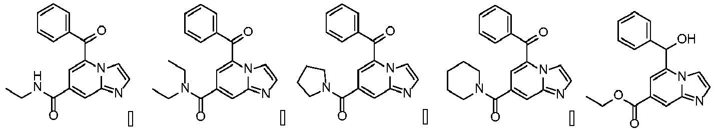

The compounds shown in the general formula (I) of the invention can be prepared by using the known synthesis methods in the art or by using the combination of the known methods in the art with the methods described in the present invention. The solvents, temperatures, and other reaction conditions provided in the present invention are exemplary and can vary according to well-known methods in the art. The compounds described in the embodiments of the present invention can be synthesized by using appropriate starting materials according to their specific structure and the methods described in the embodiments, or can be synthesized by using a method similar to that described in the embodiments.The starting materials used for synthesizing compounds in embodiments of the present invention can be prepared by known synthesis methods or similar methods documented in the literature, or obtained from commercial sources. Compounds can be further separated into their stereoisomers through well-known methods in the art, such as crystallization, chromatography, etc., as needed. The separation conditions are easily obtained by those skilled in the art through conventional technical means or limited experiments.

The second aspect of the present invention provides a method for preparing the fused heterocyclic compound (II-1-II-18),
wherein the method comprises step(1):
under alkaline conditions, M1 and 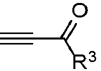 carry out the following reaction to obtain the ring fused heterocyclic compound (II-1~II-18); 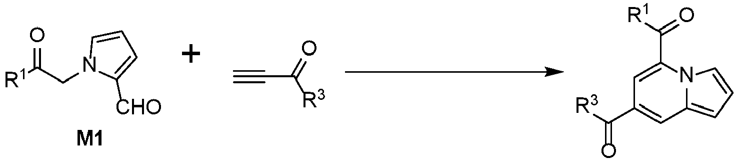
wherein R¹ is a pentamembered heterocyclic group unsubstituted, a hexamembered heterocyclic group unsubstituted, or a phenyl group unsubstituted or with at least one hydrogen atom substituted by R¹⁻², wherein R¹⁻² is a halogen, C₁₋₆ alkyl group, C₁₋₆ alkoxy group; the halogen is preferably fluorine, chlorine, or bromine; the C₁₋₆ alkyl group is preferably C₁₋₄ alkyl (such as methyl, ethyl, n-propyl, isopropyl, n-butyl, isobutyl, or tert butyl); the C₁₋₆ alkoxy group is C₁₋₄ alkoxy group (for example methoxy);
R³ is a C₁₋₄ alkyl group, or C₁₋₆ alkoxy group; the C₁₋₄ alkyl group is preferably methyl, ethyl, n-propyl, isopropyl, n-butyl, isobutyl, or tert butyl (for example methyl); the C₁₋₆ alkoxy group is preferably C₁₋₄ alkoxy group (such as methoxy, ethoxy, or propoxy).

In some preferred embodiments, in the step (1), the reaction is carried out in a polar solvent, preferably in N, N-dimethylformamide;
in the step (1), the reaction is carried out in a nitrogen atmosphere;
in the step (1), the base is a weak base, preferably sodium carbonate or potassium carbonate, for example potassium carbonate;
in the step (1), the temperature of the reaction is between 70 °C and 90 °C, for example 80 °C;
in the step (1), the reaction time is 11-13 hours, for example 12 hours;
in the step (1), the reaction further includes extraction, drying, and reduced pressure distilling to remove solvents.

In some preferred embodiments, in the step (1), the extraction is extracting with ethyl acetate and water in sequence;
in the step (1), the drying is achieved by drying with anhydrous sodium sulfate; following the step (1), the method further includes step (2): purifying the product obtained from step (1).

In some preferred embodiments, the purifying is column chromatography purification.

In some preferred embodiments, the reaction includes the steps of: under the condition of nitrogen protection, dissolving M1, 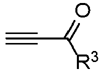 and potassium carbonate in anhydrous N, N-dimethylformamide and reacting at 80 °C for 11-13 hours, after monitoring the complete reaction of the raw materials by thin layer chromatography, cooling to room temperature, extracting the reaction solution with ethyl acetate, and extracting the aqueous phase twice with ethyl acetate, combing the organic phase, drying with anhydrous sodium sulfate, and removing the solvent by reduced pressure distillation, purifying the obtained crude product with silica gel column chromatography to obtain the fused heterocyclic compounds (II-1~II-18).

In some preferred embodiments, hydrolyzing the product obtained from the step (1) to obtain the fused heterocyclic compound (II-19) of the present invention; preferably, the hydrolyzing is carried out in an aqueous solution of sodium hydroxide.

In some preferred embodiments, hydrolyzing the product obtained from the step (1) and then adding 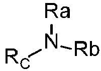 to the reaction, thus obtaining the fused heterocyclic compounds (II-20~II-36) of the present invention, wherein Ra, Rb, and Rc are independently selected from hydrogen, C₁₋₄ alkyl group (such as methyl, ethyl, n-propyl, isopropyl, n-butyl, isobutyl, or tert butyl), and C₁₋₄ alkoxy group (such as ethoxy) respectively; preferably, 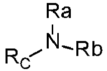 is selected from

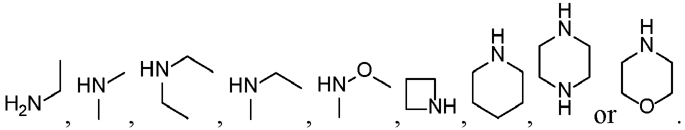

Based on the technical solution described in the present invention and common knowledge, it is known to those skilled in the art that the product obtained from the step (1) can be reacted in the presence of sodium borohydride reducing agent to obtain the fused heterocyclic compound (II-41) of the present invention.

The second aspect of the present invention also provides a method for preparing the fused heterocyclic compound(III-1~III-4), the method comprises steps (a):
under alkaline conditions, M2 and 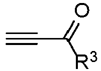 carry out the following reaction to obtain the fused heterocyclic compound; 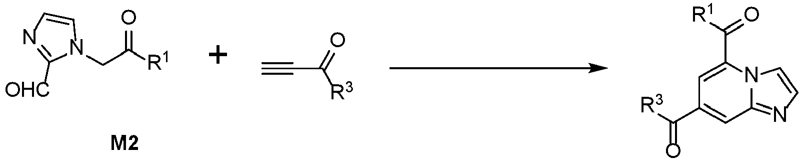
wherein R¹ is a phenyl group unsubstituted or substituted by halogen;
R³ is a hydroxyl group, C₁₋₄ alkyl group (such as methyl, ethyl, n-propyl, isopropyl, n-butyl, isobutyl, or tert butyl), or C₁₋₄ alkoxy group (such as ethoxy).

In some preferred embodiments, in the step (a), the reaction is carried out in a polar solvent, preferably in N, N-dimethylformamide;
in the step (a), the reaction is carried out in a nitrogen atmosphere;
in the step (a), the base is a weak base, preferably sodium carbonate or potassium carbonate, for example potassium carbonate;
in the step (a) the temperature of the reaction is between 70 °C and 90 °C, for example 80 °C;
in the step (a) the reaction time is 11-13 hours, for example 12 hours;
in the step (a), the reaction further includes extraction, drying, and reduced pressure distilling to remove the solvent.

In some preferred embodiments, in the step (a), the extraction is extracting with ethyl acetate and water in sequence;
in the step (a), the drying is achieved by drying with anhydrous sodium sulfate; following the step (a), the method further includes step (b): purifying the product obtained from the step (a).

In some preferred embodiments, the reaction includes the steps of: under the condition of nitrogen protection, dissolving M2 , 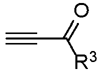 and potassium carbonate in anhydrous N, N-dimethylformamide and reacting at 80 °C for 11-13 hours, after the monitoring the complete reaction of the raw materials by thin layer chromatography, cooling to room temperature, extracting the reaction solution with ethyl acetate, and extracting the aqueous phase twice with ethyl acetate, combining the organic phases, drying with anhydrous sodium sulfate, and removing the solvent by reduced pressure distillation, purifying the obtained crude product with silica gel column chromatography to obtain the fused heterocyclic compounds (III-1~III-4).

In some preferred embodiments, hydrolyzing he product obtained from the step (a) and then adding 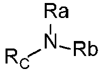 to the reaction, thus obtaining the fused heterocyclic compounds (II-20~II-36) of the present invention, wherein Ra, Rb, and Rc are independently selected from hydrogen, C₁₋₄ alkyl group (such as methyl, ethyl, n-propyl, isopropyl, n-butyl, isobutyl, or tert butyl), and C₁₋₄ alkoxy (such as ethoxy) respectively; preferably, 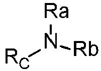 is selected from

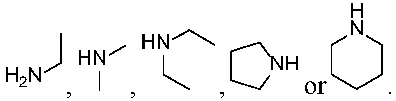

In some preferred embodiments, M1 or M2 can be prepared by the following reaction: under alkaline conditions, M1 or M2 is obtained by the reaction of α- Bromone with pyrrole-2-formaldehyde;

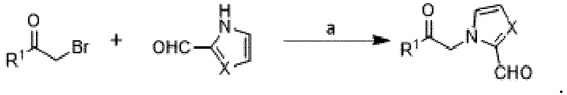

The third aspect of the present invention provides a pharmaceutical composition, wherein the pharmaceutical composition comprises the fused heterocyclic compound in the first aspect of the present invention, or a pharmaceutically acceptable salt, stereoisomer, solvate or prodrug thereof.

The fourth aspect of the present invention provides the application of a fused heterocyclic compound of the above-mentioned or a pharmaceutically acceptable salt, stereoisomer, solvate or prodrug thereof in the preparation of TRPM2 inhibitor drugs.

The fifth aspect of the present invention provides the application of pharmaceutical composition of the above-mentioned in the preparation of TRPM2 inhibitor drugs.

The sixth aspect of the of the present invention provides the application of the above-mentioned fused heterocyclic compounds or pharmaceutical compositions in the preparation of drugs for treating the TRPM2 dysfunctional diseases.

In some preferred embodiments, the TRPM2 dysfunctional diseases include: spinal cord injury or pain, cardiovascular and cerebrovascular diseases, Alzheimer's disease, neuropathic pain, Parkinson's disease, bipolar disorder or amyotrophic lateral sclerosis, liver and kidney ischemic diseases.

In some preferred embodiments, the cardiovascular and cerebrovascular diseases are caused by oxidative stress caused by ischemia/reperfusion.

In some preferred embodiments, the cardiovascular and cerebrovascular diseases include apoplexy, cerebral thrombosis, cerebral stroke, coronary heart disease, myocardial infarction, or heart failure.

The seventh aspect of the present invention provides a method for preventing and/or treating TRPM2 dysfunctional diseases, comprising the steps of: administering an effective dose of the aforementioned fused heterocyclic compound or pharmaceutical composition to patients with TRPM2 dysfunctional diseases.

The eighth aspect of the implementation method of the present invention provides a method for treating cerebral stroke , which comprises the steps of: administering an effective dose of the aforementioned fused heterocyclic compound or a pharmaceutically acceptable salt thereof to cerebral stroke patients.

The ninth aspect of the embodiment of the present invention provides a method for treating Parkinson's disease, which comprises the steps of: administering an effective dose of the aforementioned fused heterocyclic compound or a pharmaceutically acceptable salt thereof to patients with Parkinson's disease. On the basis of not violating common knowledge in the field, the above preferred conditions can be arbitrarily combined to obtain the preferred embodiments of the present invention.

The reagents and raw materials used in the present invention are commercially available.

Compared with the prior art, the embodiments of the present invention have at least the following advantages:
(1) The fused heterocyclic compound provided in the first aspect of the present invention can serve as a TRPM2 inhibitor, which helps to further elucidate the TRPM2 channel function, confirm its use as a therapeutic target for various diseases, and facilitate drug development for the target.
(2) The first aspect of the present invention provides a fused heterocyclic compound, which can be used as a TRPM2 inhibitor and apply to prepare drugs for treating TRPM2 dysfunction diseases;
(3) The present invention opens up new application fields for fused heterocyclic compounds, especially in the preparation of drugs for treating the cerebral stroke.
(4) The product of a fused heterocyclic compound of the present invention has the function of significantly reducing the area of cerebral infarction and enhancing autophagy function during ischemia-reperfusion, thereby achieving brain protection;
(5) The product of a fused heterocyclic compound of the present invention can inhibit the accumulation of zinc ions in neurons by inhibiting the TRPM2 channel, thereby achieving brain protection, therefore, the fused heterocyclic compound of the present invention facilitates the repair of brain injury;
(6) The present invention opens up new application fields for fused heterocyclic compounds, especially in the preparation of drugs for the treatment of Parkinson's disease.
(7) The product of a fused heterocyclic compound of the present invention can effectively inhibit the death of DA neurons in the SNc brain region during the progression of Parkinson's disease, alleviate damage to dopaminergic neurons derived from sporadic PD patients, and improve the vitality of differentiated dopaminergic neurons.

### Description of drawings

One or more embodiments are exemplarily illustrated by the figures in the accompanying drawings, which do not constitute a limitation of the embodiments.
FIG. 1 is a schematic diagram of electrophysiologically representative currents for inhibition of TRPM2 channel currents by the fused heterocyclic compounds II-1, II-19, and II-36 according to embodiment 66 of the present invention;
FIG. 2 is a schematic diagram of electrophysiological results of current inhibition of voltage-gated channels, NMDA receptors, ASIC channels, TRPM8 channels, TRPC6 channels, TRPV4 channels, and TRPV1 channels by the fused heterocyclic compounds II-1 according to embodiment 67 of the present invention;
FIG. 3 is a schematic diagram of the results of TTC staining of the ischemic side of mouse brain tissue by Compound II-1 and a positive control drug (Edaravone) after transient middle cerebral artery occlusion model (tMCAO) modeling of C57BL/6 mice according to embodiment 68 of the present invention;
FIG. 4 is a schematic diagram of the infarct volume of mouse brain tissue according to embodiment 68 of the present invention;
FIG. 5 is a schematic diagram of the results of scoring of neuromotor dysfunction after 24 hours of ischemia/reperfusion in various groups of model mice according to embodiment 68 of the present invention.
FIG. 6 is a schematic diagram of the results of TTC staining of the ischemic side of the brain tissue of the mice by Compound II-1 and a positive control drug (Edaravone) after transient middle cerebral artery occlusion model (tMCAO) modeling of C57BL/6 mice according to embodiment 69 of the present invention;
FIG. 7 is a schematic diagram of the infarct area of the mouse brain tissue according to embodiment 70 of the present invention;
FIG. 8 is a schematic diagram of the results of scoring of neuromotor dysfunction after 24 hours of ischemia/reperfusion in each group of model mice according to embodiment 70 of the present invention;
FIG. 9 is a schematic diagram of the results of monitoring hippocampal brain slices of wild-type and TRPM2 knockout mice by PI staining and Newport Green staining according to embodiment 70 of the present invention;
FIG. 10 is a schematic diagram of the amount of intracellular zinc ions and the number of neuronal cell deaths in wild-type and TRPM2 knockout mice after OGD according to embodiment 70 of the present invention;
FIG. 11 is a schematic diagram of the results of TTC staining, cerebral infarct area, and neuromotor dysfunction score test results on the ischemic side of brain tissue of wild-type and TRPM2 knockout mice after tMCAO modeling in embodiment 71 of the present invention;
FIG. 12 is a schematic diagram of the results of TTC staining, cerebral infarct area, and neuromotor dysfunction score test results on the ischemic side of brain tissues of wild-type and TRPM2 knockout mice administered with the autophagy inhibitor 3-MA in tMCAO modeling in embodiment 71 of the present invention.
FIG. 13 is a schematic diagram of the results of cell viability detection on dopaminergic neurons differentiated from induced pluripotent stem cells (iPSC) derived from normal person and disseminated PD patients according to embodiment 72 of the present invention;
FIG. 14 is a schematic diagram of the results of cell viability detection on dopaminergic neurons differentiated from induced pluripotent stem cells (iPSC) derived from disseminated PD patients after treating with TRPM2 inhibitor II-23 and ACA according to embodiment 72 of the present invention;
FIG. 15 is a schematic diagram of the results of after treating dopaminergic neurons differentiated from induced pluripotent stem cells (iPSCs) derived from disseminated PD patients with TRPM2 inhibitor II-23 and ACA , labeling with an susceptible dopaminergic neuron molecule Girk2 protein antibody, and mitochondrial staining with a Mitotracker, according to embodiment 72 of the present invention;
FIG. 16 is a schematic diagram of the results of mitochondrial size statistics for IPSC-induced differentiated dopaminergic neurons of FIG. 4, according to embodiment 72 of the present invention;
FIG. 17 is a schematic diagram of immunofluorescence staining of mouse striatal tyrosine hydroxylase in wild-type and TRPM2 knockout mice after fisetinone modeling according to embodiment 73 of the present invention;
FIG. 18 is a schematic diagram of the statistical results of mouse striatal tyrosine hydroxylase immunofluorescence intensity on the destroyed side compared to the undamaged side according to embodiment 73 of the present invention;
FIG. 19 is a schematic diagram of the statistical results of the rotating rod behavioral experiment according to embodiment 73 of the present invention;
FIG. 20 is a schematic diagram of the results of the first week of apomorphine-induced rotarod behavioral test according to embodiment 73 of the present invention;
FIG. 21 is a schematic diagram of the results of the second week of the apomorphine-induced rotational behavioral test according to embodiment 73 of the present invention.

### Embodiments

In order to make the objects, technical solutions and advantages of the embodiments of the present invention more clearly, the various embodiments of the present invention will be described in detail below with reference to the embodiments. However, those of ordinary skill in the art can appreciate that, in the various embodiments of the present invention, many technical details are set forth in order for the reader to better understand the present application. However, even without these technical details and various changes and modifications based on the following embodiments, the technical solutions claimed in the present application can be realized.

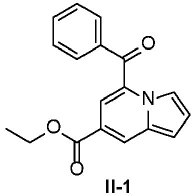

### Embodiment 1: Preparation of 5-benzoimide 7-carboxylic acid ethyl ester (II-1).

Step a1: Under nitrogen protection, dissolving pyrrole-2-carbaldehyde (0.95 g, 10.0 mmol) and potassium carbonate (1.66 g, 12.0 mmol) in 50 mL of anhydrous acetonitrile, and then adding 2-bromo-1-phenylethane-1-ketone (2.3 g, 12.0 mmol) in batches, after completely adding, heating up to 60 ° C and reacting overnight, after monitoring the complete reaction of the raw materials by thin-layer chromatography, cooling to room temperature and filtering to remove the potassium carbonate, reduced pressure distilling the filtrate to remove the solvent, and purifing the obtained crude product by silica gel column chromatography to obtain a product 1- (2-oxo-2-phenylethyl) -1H-pyrrol-2-formaldehyde , which is a white solid.

Step b: Under nitrogen protection, dissolving the compound 1- (2-oxo-2-phenylethyl) -1H-pyrrole-2-formaldehyde obtained from the previous step (852 mg, 4.0 mmol), ethyl propiolate (470 mg, 4.8 mmol), and potassium carbonate (662 mg, 4.8 mmol) in 25 mL of anhydrous N, N-dimethylformamide, and reacting overnight at 80 °C, after monitoring the complete reaction of the raw materials by thin layer chromatography, cooling to room temperature, extracting the reaction solution with 100ml and 50ml of ethyl acetate, and extracting the aqueous phase twice with ethyl acetate (50ml× 2), combining the ethyl acetate phases, drying with anhydrous sodium sulfate, and removing the solvent by reduced pressure distillation, purifying the obtained crude product by silica gel column chromatography to obtain a yellow solid (II-1, 63%).¹H NMR (500 MHz, CDCl₃): δ 8.93 (m, 1H), 8.47 (d, J = 1.5 Hz, 1H), 7.84 (dd, J = 8.0, 1.5Hz, 2H), 7.78 (d, J = 1.5 Hz, 1H), 7.66 (tt, J = 7.5, 1.5 Hz, 1H), 7.55 (t, J = 7.5 Hz, 2H), 7.10 (dd, J = 4.0, 3.0 Hz, 1H), 7.03 (dd, J = 4.0, 1.0 Hz, 1H), 4.38 (q, J = 7.0 Hz, 2H), 1.40 (t, J = 7.0 Hz, 3H); HRMS (ESI): m/z predictive value: C₁₉H₁₇NO₃ [M+H]⁺ 294.1130, detection value: 294.1132.

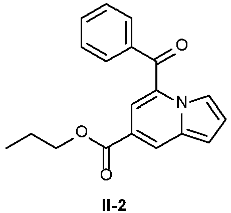

### Embodiment 2: Preparation of 5-benzoimide 7-carboxypropyl ester (II-2).

According to the method of Embodiment 1, a yellow solid (II-2, 54%) is obtained by replacing ethyl propiolate with propyl propiolate (538 mg, 4.8 mmol).

¹H NMR (500 MHz, CDCl₃): δ 8.74 (m, 1H), 8.42 (d, J = 1.0 Hz, 1H), 7.76 (dd, J = 7.5, 2.0 Hz, 2H), 7.64 (d, J = 2.0 Hz, 1H), 7.53 (tt, J = 7.5, 1.5 Hz, 1H), 7.47 (t, J = 7.5 Hz, 2H), 7.08 (dd, J = 4.0, 3.0 Hz, 1H), 7.01 (dd, J = 4.0, 1.0 Hz, 1H), 4.33 (q, J = 7.0 Hz, 2H), 1.85 (m, 2H), 1.04 (t, J = 7.0 Hz, 3H);HRMS (ESI): m/z predictive value: C₁₈H₁₅NO₃ [M+H]⁺ 308.1287, detection value: 308.1295.

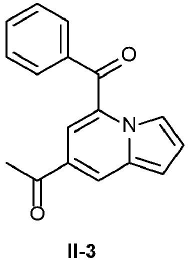

### Embodiment 3: Preparation of 1-(5-Benzimide-7-yl) ethane-1-ketone (II-3) .

According to the method of Embodiment!, a yellow solid (II-3, 57%) is obtained by replacing ethyl propiolate with propane-3-alkyne-2-ketone (326mg, 4.8mmol).1H NMR (500 MHz, CDC13): δ 8.87 (m, 1H), 8.31 (m, 1H), 7.81 (dt, J = 7.5, 2.0 Hz, 2H), 7.76 (d, J = 2.0 Hz, 1H), 7.64 (tt, J = 7.0, 1.0 Hz, 1H), 7.53 (tt, J = 7.0, 2.0 Hz, 2H), 7.08 (dd, J = 4.5, 3.0 Hz, 1H), 7.05 (dd, J = 4.5, 1.0 Hz, 1H), 2.58 (s, 3H); HRMS (ESI): m/z predictive value: C18H15NO3 [M+H] + 264.1025, detection value: 264.1030.

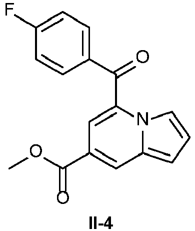

### Embodiment 4: Preparation of 5- (4-Fluorobenzoyl) indolizine-7-methyl carboxylate (II-4).

Step a2: Under nitrogen protection, dissolving pyrrole-2-carbaldehyde in anhydrous N, N-dimethylformamide, cooling the reaction solution to 0°Cby ice bath, then adding sodium hydride in batches(0.29g, 12.0mmol), after completely adding, reacting at 30°C for 30 mins, dropwisely adding 2-bromo 1- (4-fluorophenyl) ethane-1-ketone dissolved in N, N-dimethylformamide (2.6g, 12.0mmol), after dropwisely adding heating up to room temperature and reacting overnight, after monitoring the complete reaction of the raw materials by thin-layer chromatography, dropwisely adding saturated oxidation solution into the reaction solution under ice bath, after dropwisely adding, extracting the reaction solution with ethyl acetate and water, and extracting the aqueous phase twice with ethyl acetate, combining the ethyl acetate phases, drying with anhydrous sodium sulfate, and removing the solvent by reduced pressure distillation, purifying the obtained crude product by silica gel column chromatography to obtain a product, which is a white solid, and directly applied in next reaction, the method is applicable to the synthesis of intermediate M1 that R1 is a benzene ring, furan ring, thiophene ring and pyridine ring substituted by fluorine.

According to the method of Embodiment 2, a yellow solid (II-4, 45%) is obtained by replacing 2-bromo-1-phenylethane-1-ketone with 2-bromo-1- (4-fluorophenyl) ethane-1-ketone (2.6g, 12.0mmol) and replacing ethyl propiolate with methyl propiolate (403mg, 4.8mmol). 1H NMR (500 MHz, CDC13): δ 8.86 (d, J = 2.5 Hz, 1H), 8.47 (d, J = 1.5 Hz, 1H), 7.89 (td, J = 5.5, 2.0 Hz, 2H), 7.71 (d, J = 1.5 Hz, 1H), 7.24 (t, J = 7.5 Hz, 2H), 7.10 (q, J = 7.0 Hz, 1H,), 7.04 (dd, J = 4.0, 1.0 Hz, 1H), 3.92 (s, 3H); HRMS (ESI): m/z predictive value: C17H12FNO3 [M+H] + 298.0897, detection value: 298.0899.

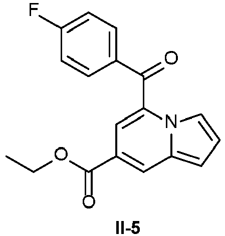

Embodiment 5: Preparation of 5- (4-fluorobenzoyl) indolizinee-7-ethyl carboxylate (II-5).

According to the method of Embodiment 4, a yellow solid (II-5, 56%) is obtained by replacing ethyl propiolate with methyl propiolate (346mg, 4.8mmol) .1H NMR (500 MHz, CDC13): δ 8.86 (d, J = 2.5 Hz, 1H), 8.47 (J = 1.5 Hz, 1H), 7.88 (td, J = 5.5, 2.0 Hz, 2H), 7.73 (J = 1.5 Hz, 1H), 7.24 (t, J = 8.5 Hz, 2H), 7.10 (dd, J = 4.5, 2.0 Hz, 1H), 7.03 (dd, J = 4.5, 1.5 Hz, 1H), 4.39 (q, J = 7.0 Hz, 2H), 1.40 (t, J = 7.0 Hz, 3H); HRMS (ESI): m/z predictive value: C18H14FNO3 [M+H] + 312.1036, detection value: 312.1038.

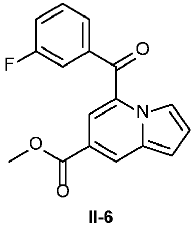

Embodiment 6: Preparation of 5- (3-Fluorobenzoyl)indolizine-7-methyl carboxylate ( II-6).

According to the method of Embodiment 4, a yellow solid (II-6, 61%) is obtained by replacing 2-bromo-1- (4-fluorophenyl) ethane-1-ketone with 2-bromo-1- (3-fluorophenyl) ethane-1-ketone (2.6 g, 12.0 mmol). 1H NMR (500 MHz, CDC13): δ 8.95 (d, J = 2.0 Hz, 1H), 8.50 (d, J = 1.5 Hz, 1H), 7.77 (d, J = 1.5 Hz, 1H), 7.60 (d, J = 7.5 Hz, 1H), 7.54 (m, 2H), 7.36 (tdd, J = 8.5, 2.5, 1.0 Hz, 1H), 7.12 (t, J = 6.5 Hz, 1H), 7.06 (dd, J = 5.5 Hz, 1H), 3.93 (s, 3H); HRMS (ESI): m/z predictive value: C17H12FNO3 [M+H] + 298.0897, detection value: 298.0901.

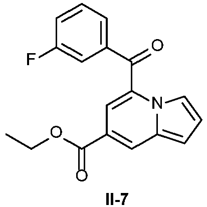

### Embodiment 7 : Preparation of 5-(3-fluorobenzoyl)indolizine-7-ethyl carboxylate (II-7) .

According to the method of Embodiment 4, a yellow solid (II-7, 58%) is obtained by replacing 2-bromo-1- (4-fluorophenyl) ethane-1-ketone with 2-bromo-1- (3-fluorophenyl) ethane-1-ketone (2.6 g, 12.0 mmol) and replacing methyl propiolate with ethyl propiolate (403 mg, 4.8 mmol). 1H NMR (500 MHz, CDC13): δ 8.95 (d, J = 2.5 Hz, 1H), 8.49 (d, J = 1.5 Hz, 1H), 7.79 (d, J = 1.5 Hz, 1H), 7.60 (dt, J = 7.5, 1.0 Hz, 1H), 7.54 (m, 1H), 7.36 (tdd, J = 8.0, 2.5 Hz, 1H), 7.12 (t, J = 7.0 Hz, 1H), 7.06 (dd, J = 4.5, 1.0 Hz, 1H), 4.39 (q, J = 7.0 Hz, 2H), 1.40 (t, J = 7.0 Hz, 3H); HRMS (ESI): m/z predictive value: C17H12FNO3 [M+H] + 312.1036, detection value: 312.1039.

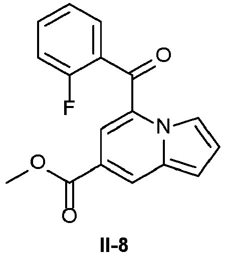

### Embodiment 8: Preparation of 5-(2-Fluorobenzoyl) indolizine-7-methyl carboxylate (II-8) .

According to the method of Embodiment 4, a yellow solid (II-8, 47%) is obtained by replacing 2-bromo-1-(4-fluorophenyl)ethane-1-ketone with 2-bromo-1- (2-fluorophenyl) ethane-1-ketone (2.6 g, 12.0 mmol) . 1H NMR (500 MHz, CDC13): δ 9.24 (d, J = 2.0 Hz, 1H), 8.51 (d, J = 1.0 Hz, 1H), 7.79 (t, J = 1.5 Hz, 1H), 7.59 (m, 2H), 7.33 (t, J = 1.5 Hz, 1H), 7.24 (t, J = 9.0 Hz, 1H), 7.15 (dd, J = 4.0, 2.5 Hz, 1H), 7.08 (d, J = 4.0 Hz, 1H), 3.90(s, 3H); HRMS (ESI): m/z, predictive value: C17H12FNO3 [M+H] + 298.0897, detection value: 298.0898.

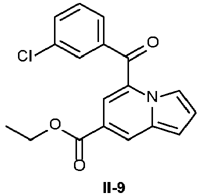

### Embodiment 9: Preparation of 5- (3-chlorobenzoyl) indolizine-7-ethyl carboxylate (II-9).

According to the method of Embodiment 1, a yellow solid (II-9, 71%) is obtained by replacing 2-bromo-1-phenylethane-1-ketone with 2-bromo-1- (3-chlorophenyl) ethane-1-ketone (2.8 g, 12.0 mmol). 1HNMR (500 MHz, CDC13): δ 8.74 (d, J = 2.0 Hz, 1H), 8.26 (d, J = 2.0 Hz, 1H), 7.57 (d, J = 1.5 Hz, 1H), 7.42 (dd, J = 7.5, 1.5 Hz, 1H), 7.36 (m, 2H), 7.23 (dd, J = 4.0, 3.0 Hz, 1H), 7.12 (t, J = 7.0 Hz, 1H), 7.03 (dd, J = 4.5, 1.0 Hz, 1H), 4.17 (q, J = 7.0 Hz, 2H), 1.41 (t, J = 7.0 Hz, 3H); HRMS (ESI): m/z predictive value: C17H12C1NO3 [M+H] + 328.0740, detection value: 328.0745.

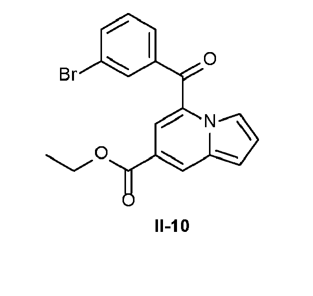

### Embodiment 10 : Preparation of 5- (3-bromobenzoyl) indolizine-7-Ethyl carboxylate (II-10).

According to the method 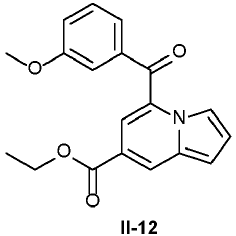 of Embodiment 1, a yellow solid (II-10, 65%) is obtained by replacing 2-bromo-1-phenylethane-1-ketone with 2-bromo-1-(3-bromophenyl) ethane-1-ketone (3.3 g, 12.0 mmol) . 1H NMR (500 MHz, CDC13): δ 8.72 (d, J = 1.5 Hz, 1H), 8.24 (d, J = 2.0 Hz, 1H), 7.56 (m, 1H), 7.41 (dd, J = 7.0, 2.0 Hz, 1H), 7.34 (m, 2H), 7.21 (dd, J = 4.0, 3.0 Hz, 1H), 7.09 (t, J = 7.0 Hz, 1H), 7.01 (dd, J = 4.5, 1.0 Hz, 1H), 4.18 (q, J = 7.0 Hz, 2H), 1.40 (t, J = 7.0 Hz, 3H); HRMS (ESI): m/z predictive value: C17H12BrNO3 [M+H] + 372.0235, detection value: 372.0238.

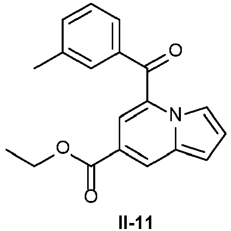

### Embodiment 11: Preparation of 5- (3-methylbenzoyl) indolizine-7-Ethyl carboxylate (II-11).

According to the method of Embodiment 1,a yellow solid (II-11, 74%) is obtained by replacing 2-bromo-1-phenylethane-1-ketone with 2-bromo-1- (m-methylphenyl) ethane-1-ketone (2.5 g, 12.0 mmol) . 1H NMR (500 MHz, CDC13): δ 8.61 (m, 1H), 8.18 (d, J = 2.0 Hz, 1H), 7.49 (m, 1H), 7.35 (m, 3H), 7.17 (dd, J = 4.0, 3.0 Hz, 1H), 7.08 (t, J = 7.0 Hz, 1H), 7.02 (dd, J = 4.5, 1.5 Hz, 1H), 4.21 (q, J = 7.0 Hz, 2H), 1.41 (t, J = 7.0 Hz, 3H); HRMS (ESI): m/z predictive value: C18H15NO3 [M+H] + 308.1287, detection value: 308.1285.

### Embodiment 12: Preparation of Ethyl 5- (3-methoxybenzoyl) indolizine-7-carboxylate (II-12).

According to the method of Embodiment 1, a yellow solid (II-12, 78%) is obtained by replacing 2-bromo-1-phenylethane-1-ketone with 2-bromo-1- (3-methoxyphenyl) ethane-1-ketone (2.7 g, 12.0 mmol). 1H NMR (500 MHz, CDC13): δ 8.67 (d, J = 2.0 Hz, 1H), 8.18 (d, J = 1.5 Hz, 1H), 7.47 (m, 1H), 7.36 (m, 2H), 7.21 (dd, J = 4.0, 3.0 Hz, 1H), 7.13 (t, J = 7.0 Hz, 1H), 7.04 (dd, J = 4.5, 1.0 Hz, 1H), 4.20 (q, J = 7.0 Hz, 2H), 3.64 (s, 3H), 1.41 (t, J = 7.0 Hz, 3H); HRMS (ESI): m/z predictive value: C19H17NO4 [M+H] + 324.1236, detection value: 324.1238.

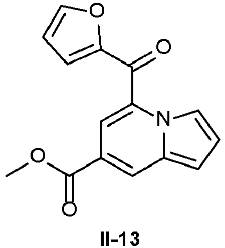

### Embodiment 13: Preparation of 5- (furan-2-carbonyl) indolizine-7-methyl carboxylate (II-13).

According to the method of Embodiment 1, a yellow solid (11-13, 65%) is obtained by replacing 2-bromo-1-phenylethane-1-ketone with 2-bromo-1-(furan-2-yl) ethane-1-ketone (2.3 g, 12.0 mmol) and replacing ethyl propiolate with methyl propiolate (403 mg, 4.8 mmol). 1H NMR (500 MHz, CDC13): δ 8.79 (d, J = 3.0 Hz, 1H), 8.47 (d, J = 1.0 Hz, 1H), 8.19 (d, J = 1.5 Hz, 1H), 7.80 (dd, J = 1.5, 0.5 Hz, 1H), 7.35 (dd, J = 3.5, 0.5 Hz, 1H), 7.07 (dd, J = 4.5, 3.0 Hz, 1H), 7.01 (J = 4.5, 1.0 Hz, 1H), 6.68 (dd, J = 4.0, 2.0 Hz, 1H), 3.97 (s, 3H); HRMS (ESI): m/z predictive value: C15H11NO4 [M+H] + 270.0766, detection value: 270.0768.

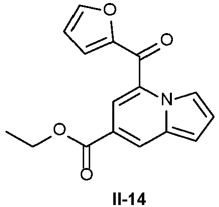

### Embodiment 14: Preparation of 5- (furan-2-carbonyl) indolizine-7-ethyl carboxylate (II-14)

According to the method of Embodiment 1, a yellow solid (11-14, 57%) is obtained by replacing 2-bromo-1-phenylethane-1-ketone with 2-bromo-1-(furan-2-yl) ethane-1-ketone (2.3g, 12.0 mmol) . 1H NMR (500 MHz, CDC13): δ 8.72 (d, J = 3.0 Hz, 1H), 8.45 (d, J = 1.0 Hz, 1H), 8.14 (d, J = 1.5 Hz, 1H), 7.79 (dd, J = 1.5, 0.5 Hz, 1H), 7.42 (dd, J = 3.5, 0.5 Hz, 1H), 7.14 (dd, J = 4.5, 3.0 Hz, 1H), 6.94 (J = 4.5, 1.0 Hz, 1H), 6.65 (dd, J = 4.0, 2.0 Hz, 1H), 4.37 (q, J = 7.0 Hz, 2H), 1.49 (t, J = 7.0 Hz, 3H); HRMS (ESI): m/z predictive value: C16H13NO4 [M+H] + 284.0923, detection value: 284.0927.

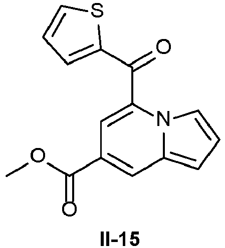

### Embodiment 15 : Preparation of 5-(thiophen-2-carbonyl)indolizine-7-methyl carboxylate (11-15).

According to the method of Embodiment 1, a yellow solid (11-15, 78%) is obtained by replacing 2-bromo-1-phenylethane-1-ketone with 2-bromo-1-(thiophen-2-yl) ethane-1-one (2.4 g, 12.0 mmol) and replacing ethyl propiolate with methyl propiolate (403 mg, 4.8 mmol). 1H NMR (500 MHz, CDC13): δ 8.65 (m, 1H), 8.46 (d, J = 1.5 Hz, 1H), 7.99 (d, J = 1.5 Hz, 1H), 7.81 (dd, J = 5.0, 1.0 Hz, 1H), 7.78 (dd, J = 4.0, 1.0 Hz, 1H), 7.24 (dd, J = 5.0, 4.0 Hz, 1H), 7.06 (dd, J = 4.5, 3.0 Hz, 1H), 7.00 (dd, J = 4.5, 1.0 Hz, 1H), 3.96 (s, 3H); HRMS (ESI): m/z predictive value: C15H11NO3S [M+H] + 286.0538, detection value: 286.0540.

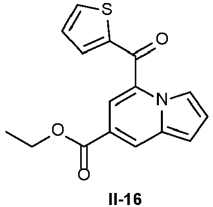

### Embodiment 16: Preparation of 5-(thiophen-2-carbonyl) indolizine-7-ethyl carboxylate (II-16).

According to the method of Embodiment 1, a yellow solid (11-16, 76%) is obtained by replacing 2-bromo-1-phenylethane-1-ketone with 2-bromo-1-(thiophen-2-yl) ethane-1-ketone (2.4 g, 12.0 mmol). 1H NMR (500 MHz, CDC13): δ 8.59 (m, 1H), 8.37 (d, J = 1.5 Hz, 1H), 7.91 (d, J = 1.5 Hz, 1H), 7.83 (dd, J = 4.5, 1.5 Hz, 1H), 7.75 (dd, J = 4.0, 1.5 Hz, 1H), 7.21 (dd, J = 4.5, 4.0 Hz, 1H), 7.03 (dd, J = 4.5, 3.0 Hz, 1H), 6.97 (dd, J = 4.5, 1.5 Hz, 1H), 4.35 (q, J = 7.0 Hz, 2H), 1.47 (t, J = 7.0 Hz, 3H); HRMS (ESI): m/z predictive value: C16H13NO3S [M+H] + 300.0694, detection value: 300.0697.

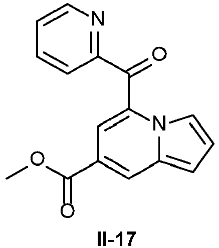

### Embodiment 17 : Preparation of 5-methylpyridinimide-7-methyl carboxylate (II-17).

According to the method of Embodiment1, a yellow solid (II-17, 78%) is obtained by replacing 2-bromo-1-phenylethane-1-ketone with 2-bromo-1-(pyridin-2-yl) ethane-1-ketone (2.3 g, 12.0 mmol) and replacing ethyl propiolate with methyl propiolate (403 mg, 4.8 mmol). 1H NMR (500 MHz, CDC13): 9.13 (m, 1H), 8.82 (m, 1H), 8.56 (d, J = 1.5 Hz, 1H), 8.23 (d, J = 1.5 Hz, 1H), 8.04 (dt, J = 7.5, 1.5 Hz, 1H), 7.97 (ddd, J = 9.0, 7.0, 1.0 Hz, 1H), 7.54 (ddd, J = 8.5, 7.0, 1.0 Hz, 1H), 7.10 (dd, J = 4.5, 2.5 Hz, 1H), 7.03 (dd, J = 4.5, 1.0 Hz, 1H), 4.05 (s, 3H); HRMS (ESI): m/z predictive value: C16H12N2O3 [M+H] + 281.0926, detection value: 281.0929.

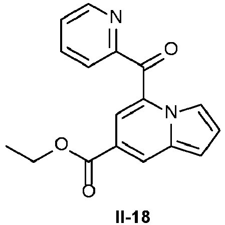

### Embodiment 18: Preparation of 5-methylpyridinimide-7-ethyl carboxylate (II-18).

According to the method of Embodiment 1, a yellow solid (11-18, 76%) is obtained by replacing 2-bromo-1-phenylethane-1-ketone with 2-bromo-1-(38yridine-2-yl) ethane-1-ketone (2.3 g, 12.0 mmol) 1H NMR (500 MHz, CDC13): δ 9.07 (m, 1H), 8.74 (m, 1H), 8.46 (d, J = 1.5 Hz, 1H), 8.21 (d, J = 1.5 Hz, 1H), 8.00 (dt, J = 8.0, 1.0 Hz, 1H), 7.93 (ddd, J = 9.0, 7.5, 1.5 Hz, 1H), 7.51 (ddd, J = 8.0, 6.5, 1.5 Hz,1H), 7.08 (dd, J = 4.5, 2.5 Hz, 1H), 7.01 (dd, J = 4.5, 1.0 Hz, 1H), 4.36 (q, J = 7.0 Hz, 2H), 1.36 (t, J = 7.0 Hz, 3H); HRMS (ESI): m/z predictive value: C17H14N2O3 [M+H] + 295.1083, detection value: 295.1088.

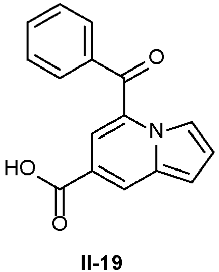

### Embodiment 19: Preparation of 5-benzimide-7-carboxylic acid (11-19).

Step c: Dissolving compound II-1 (2.9g, 10 mmol) in 30 ml of ethanol, adding 10 ml of 2N sodium hydroxide solution, and the reaction solution reacting overnight at 80 °C, after monitoring the complete reaction of the raw materials by thin-layer chromatography, removing the ethanol by reduced pressure distillation, adjusting the remaining solution to acidity with hydrochloric acid solution, and a yellow solid precipitating, after filtrating, washing the solid with water and drying to obtain a yellow solid (11-19, 95%).1H NMR (500 MHz, CDC13): δ 12.05 (s, 1H), 8.74 (d, J = 2.0 Hz, 1H), 8.35 (J = 2.0 Hz, 1H), 7.57 (m, 3H), 7.35 (dd, J = 7.5, 2.0 Hz, 1H), 7.21 (t, J = 8.5 Hz, 2H), 7.10 (dd, J = 4.5, 2.5 Hz, 1H), 7.03 (dd, J = 4.5, 1.5 Hz, 1H); HRMS (ESI): m/z predictive value: C16H11NO3 [M+H] + 266. 0817, detection value: 266.1818.

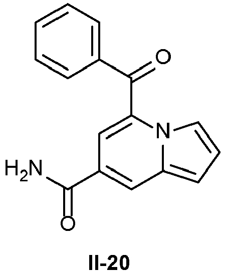

### Embodiment 20: Preparation of 5-benzimide-7-formamide (II-20)

Step d: Dissolving compound 11-19 (275mg, 1mmol), 1-hydroxybenzotriazole (64mg, 0.3mmol), and 1-ethyl - (3-dimethylaminopropyl) carbodiimide hydrochloride (229mg, 1.2mmol) in 10 mL of anhydrous tetrahydrofuran, adding N, N-diisopropylethylamine (168mg, 1.3mmol) at room temperature and reacting for 8 hours at room temperature until the complete reaction is monitored by thin layer chromatography, then adding ammonium chloride (107mg, 2mmol) and reacting for 2 hours at room temperature, after monitoring the complete reaction by thin layer chromatography, removing the solvent by reduced pressure distilling the reaction solution, and purifying the obtained crude product by silica gel column chromatography to obtain a red solid (II-20, 87%). 1H NMR (500 MHz, d6-DMSO): δ 8.75 (s, 1H), 8.50 (s, 1H), 8.10 (s, 1H), 7.82 (d, J = 7.0 Hz, 2H), 7.72 (t, J = 7.5 Hz, 1H), 7.62 (m, 3H), 7.44 (s, 1H), 7.11 (t, J = 3.5 Hz, 1H), 7.02 (d, J = 4.0 Hz, 1H); HRMS (ESI): m/z predictive value: C16H12N2O2 [M+H] + 265.0977, detection value: 265.0979.

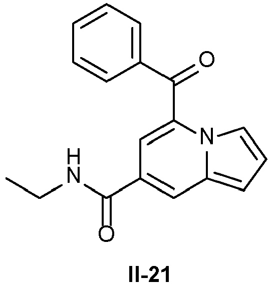

### Embodiment 21: Preparation of 5-benzoyl-N-ethylindolizine-7-formamide (II-21).

According to the method of Embodiment 20, a yellow solid (II-21, 86%) is obtained by replacing ammonium chloride with ethylamine (90mg, 2.0 mmol). 1H NMR (500 MHz, CDC13): δ 8.82 (d, J = 2.0 Hz, 1H), 8.01 (d, J = 1.5 Hz, 1H), 7.81 (dd, J = 8.0, 1.0 Hz, 2H), 7.62 (m, 2H), 7.52 (t, J = 7.5 Hz, 2H),7.05 (dd, J = 4.0, 2.5 Hz, 1H), 6.91 (dd, J = 3.5, 1.0 Hz, 1H), 6.01 (s, 1H), 3.47 (m, 2H), 1.24 (t, J = 7.0 Hz, 3H); HRMS (ESI): m/z predictive value: C18H16N2O2 [M+H] + 293.1290, detection value: 293.1292.

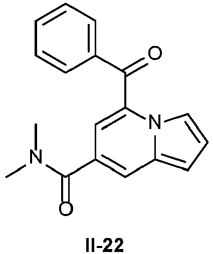

### Embodiment 22 : Preparation of 5-benzoyl-N, N-dimethylindolizine-7-formamide (II-22)

According to the method of Embodiment 20, a yellow solid (II-22, 85%) is obtained by replacing ammonium chloride with dimethylamine (90mg, 2.0 mmol).1H NMR (500 MHz, CDC13): δ 8.82 (d, J = 2.0 Hz, 1H), 7.82 (d, J = 1.5 Hz, 1H), 7.79 (dd, J = 7.0, 1.5 Hz, 2H), 7.60 (tt, J = 7.5, 1.0 Hz,1H), 7.50 (t, J = 8.0 Hz, 2H), 7.29 (d, J = 1.5 Hz, 1H), 7.00 (dd, J = 4.0, 2.5 Hz, 1H), 6.86 (dd, J = 4.5, 1.0 Hz, 1H), 3.08 (s, 6H). HRMS (ESI): m/z predictive value: C18H16N2O2 [M+H] + 293.1290, detection value: 293.1292.

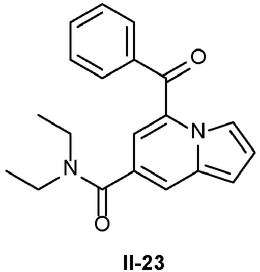

### Embodiment 23 : Preparation of 5-Benzoyl-N, N-diethylindolizine-7-formamide (II-23)

According to the method of Embodiment 20, a yellow solid (II-23, 89%) is obtained by replacing ammonium chloride with diethylamine (146mg, 2 mmol) . 1H NMR (500 MHz, CDC13): δ 8.81 (d, J = 3.0 Hz, 1H), 7.79 (m, 3H), 7.61 (tt, J = 7.5, 1.0 Hz, 1H), 7.50 (t, J = 8.0 Hz, 2H), 7.23 (d, J = 2.0 Hz, 1H), 7.04 (dd, J = 4.5, 3.0 Hz, 1H), 6.84 (dd, J = 4.5, 1.5 Hz, 1H), 3.43 (m, 4H), 1.19 (m, 6H); HRMS (ESI): m/z predictive value: C20H20N2O2 [M+H] + 321.1603, detection value: 321.1606.

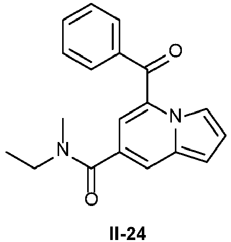

### Embodiment 24: Preparation of 5-Benzoyl-N-ethyl-N-methylindolizine-7-formamide (II-24)

According to the method of Embodiment 20, a yellow solid (II-24, 88%) is obtained by replacing ammonium chloride with methylethylamine (118mg, 2 mmol) 1NMR (500 MHz, CDC13): δ 8.84 (d, J = 1.5 Hz, 1H), 7.82 (m, 3H), 7.63 (t, J = 8.0 Hz, 1H), 7.53 (t, J = 8.0 Hz, 2H), 7.29 (m, 1H), 7.08 (dd, J = 4.0, 3.0 Hz, 1H), 6.88 (dd, J = 3.5, 1.0 Hz, 1H), 3.49 (m, 2H), 3.06 (m, 3H), 1.20 (m, 6H); HRMS (ESI): m/z predictive value: C19H18N2O2 [M+H] + 307.1447, detection value: 307.1449.

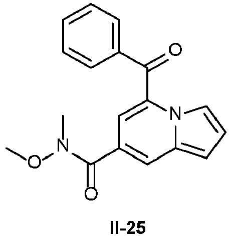

### Embodiment25: Preparation of 5-Benzoyl-N-methoxy-N-methylindolizine-7-formamide (II-25)

According to the method of Embodiment 20, a yellow solid (II-25, 85%) is obtained by replacing ammonium chloride with N, O-dimethylhydroxylamine (122mg, 2 mmol) . 1NMR (500 MHz, CDC13): δ 8.82 (d, J = 1.5 Hz, 1H), 7.79 (m, 3H), 7.58 (t, J = 8.0 Hz, 1H), 7.49 (t, J = 8.0 Hz, 2H), 7.27 (m, 1H), 7.05 (dd, J = 4.5, 3.0 Hz, 1H), 6.85 (dd, J = 4.5, 1.5 Hz, 1H), 3.59 (s, 2H), 3.36 (s, 3H); HRMS (ESI): m/z predictive value: C18H16N2O3 [M+H] + 309.1239, detection value: 209.1243.

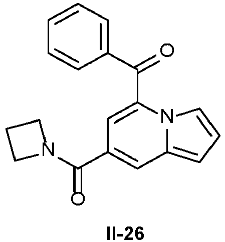

### Embodiment 26: Preparation of azetidine-1-yl (5-benzoimide 7-yl) ketone (II-26)

According to the method of Embodiment 20, a yellow solid (II-26, 77%) is obtained by replacing ammonium chloride with azetidine (122mg, 2 mmol). 1H NMR (500 MHz, CDC13): δ 8.91 (d, J = 2.0 Hz, 1H), 7.93 (d, J = 2.0 Hz, 1H), 7.87 (dd, J = 7.5, 2.0 Hz, 2H), 7.67 (tt, J = 7.5, 1.0 Hz, 1H), 7.54 (t, J = 7.5 Hz, 2H), 7.33 (d, J = 2.0 Hz, 1H), 7.07 (dd, J = 4.0, 2.5 Hz, 1H), 6.92 (dd, J = 4.5, 1.5 Hz, 1H), 4.06 (m, 4H), 3.58 (m, 2H); HRMS (ESI): m/z predictive value: C19H16N2O2 [M+H] + 305.1290, detection value: 305.1294.

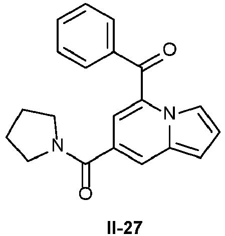

### Embodiment 27: Preparation of (5-benzoimide 7-yl) (pyrrolidine-1-yl) ketone (II-27)

According to the method of Embodiment 20, a yellow solid (II-27, 82%) is obtained by replacing ammonium chloride with pyrrolidine (142mg, 2 mmol) . 1H NMR (500 MHz, CDC13): δ 8.84 (d, J = 2.0 Hz, 1H), 7.81 (d, J = 2.0 Hz, 1H), 7.77 (dd, J = 8.0, 1.5 Hz, 2H), 7.59 (t, J = 7.5 Hz, 1H), 7.43 (t, J = 8.0 Hz, 2H), 7.29 (d, J = 2.0 Hz, 1H), 7.04 (dd, J = 4.0, 2.5 Hz, 1H), 6.87 (dd, J = 4.5, 1.0 Hz, 1H), 3.94 (m, 4H), 2.17 (m, 4H); HRMS (ESI): m/z predictive value: C20H18N2O2 [M+H] + 319.1447, detection value: 319.1451.

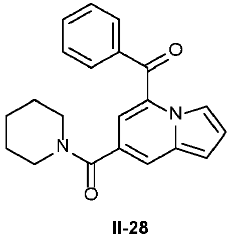

### Embodiment 28: Preparation of (5-benzimide 7-yl) (piperidin-1-yl) ketone (II-28)

According to the method of Embodiment 20, a yellow solid (II-28, 84%) is obtained by replacing ammonium chloride with piperidine (170mg, 2 mmol). 1H NMR (500 MHz, CDC13): δ 8.79 (d, J = 2.0 Hz, 1H), 7.76 (d, J = 1.5 Hz, 1H), 7.62 (dd, J = 7.5, 2.0 Hz, 2H), 7.54 (t, J = 7.5 Hz, 1H), 7.40 (t, J = 7.5 Hz, 2H), 7.25 (m, 1H), 7.01 (dd, J = 4.0, 2.5 Hz, 1H), 6.84 (dd, J = 4.5, 1.5 Hz, 1H), 3.87 (m, 4H), 1.93 (m, 4H), 1.52 (m, 2H); HRMS (ESI): m/z predictive value: C21H20N2O2 [M+H] + 333.1603, detection value: 333.1608.

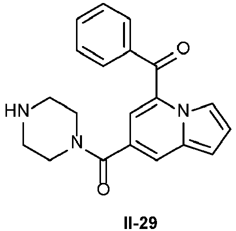

### Embodiment 29: Preparation of (5-benzoyl indolizine-7-yl) (piperazin-1-yl) ketone (II-29)

According to the method of Embodiment 20, a yellow solid (II-29, 85%) is obtained by replacing ammonium chloride with piperazine (172mg, 2 mmol) . 1NMR (500 MHz, CDC13): δ 8.81 (d, J = 2.0 Hz, 1H), 7.76 (m, 3H), 7.54 (t, J = 7.5 Hz, 1H), 7.43 (t, J = 7.5 Hz, 2H), 7.24 (d, J = 1.5 Hz, 1H), 7.03 (dd, J = 4.5, 3.0 Hz, 1H), 6.82 (dd, J = 4.5, 1.0 Hz, 1H), 4.01 (s, 1H), 3.62 (m, 4H), 3.47 (m, 4H); HRMS (ESI): m/z predictive value: C20H19N3O2 [M+H] + 334.1556, detection value: 334.1558.

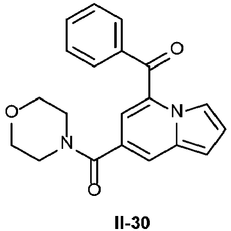

### Embodiment 30: Preparation of (5-benzimide-7-yl) (morphyryl) ketone (II-30)

According to the method of Embodiment 20, a yellow solid (11-30, 81%) is obtained by replacing ammonium chloride with morpholine (174mg, 2 mmol) .1NMR (500 MHz, CDC13): δ 8.82 (d, J = 1.5 Hz, 1H), 7.80 (d, J = 2.0 Hz, 1H), 7.68 (dd, J = 7.5, 2.0 Hz, 2H), 7.60 (t, J = 8.0 Hz, 1H), 7.45 (t, J = 8.0 Hz, 2H), 7.25 (d, J = 2.0 Hz, 1H), 7.07 (dd, J = 4.0, 2.5 Hz, 1H), 6.91 (dd, J = 4.5, 1.0 Hz, 1H), 4.05 (m, 4H), 3.93 (m, 4H); HRMS (ESI): m/z predictive value: C20H18N2O3 [M+H] + 335.1396, detection value: 335.1399.

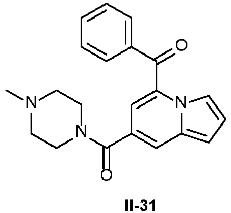

### Embodiment 31: Preparation of (5-Benzimide-7-yl) (4-methylpiperazin-1-yl) ketone (II-31)

According to the method of Embodiment 20, a yellow solid (II-31, 85%) is obtained by replacing ammonium chloride with 4-methylpiperazine (200mg, 2 mmol) δ 8.91 (d, J = 2.0 Hz, 1H), 7.87 (d, J = 1.5 Hz, 1H), 7.78 (dd, J = 8.0, 2.0 Hz, 2H), 7.69 (t, J = 7.5 Hz, 1H), 7.52 (t, J = 7.5 Hz, 2H), 7.31 (m, 1H), 7.11 (dd, J = 4.0, 2.5 Hz, 1H), 6.93 (dd, J = 4.5, 1.5 Hz, 1H), 3.72 (m, 4H), 2.93 (m, 4H), 2.78 (s, 3H); HRMS (ESI): m/z predictive value: C21H21N3O2 [M+H] + 348.1712, detection value: 348.1712.

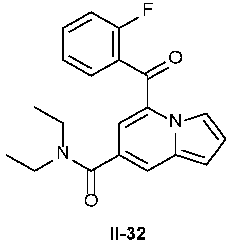

### Embodiment 32 : Preparation of N, N-Diethyl-5- (2-fluorobenzoyl) indolizine-7-formamide (II-32)

Step c: Dissolving compound II-8 (1.5g, 5mmol) in 15 mL of ethanol, adding 5 mL of 2N sodium hydroxide solution, and reacting overnight at 80 °C, after monitoring the complete reaction by thin layer chromatography, removing the ethanol by reduced pressure distillation, and adjusting the remaining solution to slight acidity with hydrochloric acid solution, and a yellow solid precipitating. After filtering washing the solid with water, and drying to obtain a yellow solid (1.3g, 94%).

Step d: Dissolving the yellow solid (283mg, 1mmol) obtained from the previous step, 1-hydroxybenzotriazole (64mg, 0.3mmol) and 1-ethyl -(3-dimethylaminopropyl) carbodiimide hydrochloride (229mg, 1.2mmol) in 10ml of anhydrous tetrahydrofuran, adding N, N-diisopropylethylamine (168mg, 1.3mmol) at room temperature and reacting for 8 hours until the complete reaction of the raw materials is monitored by thin-layer chromatography, then adding diethylamine (146mg, 2 mmol) and reacting at room temperature for 2 hours, after monitoring the complete reaction of the the raw materials by thin-layer chromatography, removing the solvent by reduced pressure distilling the reaction solution, and purifying the obtained crude product by silica gel column chromatography to obtain a red solid (II-32, 87%). 1H NMR (500 MHz, CDC13): δ 8.84 (m, 1H), 7.81 (d, J = 1.5 Hz, 1H), 7.55 (dt, J = 7.5, 1.0 Hz, 1H), 7.49 (m, 2H), 7.30 (dddd, J = 11.0, 7.5, 3.0, 1.5 Hz, 1H), 7.26 (d, J = 2.0 Hz, 1H), 7.05 (dd, J = 4.5, 3.0 Hz, 1H), 6.86 (dd, J = 4.5, 1.5 Hz, 1H), 3.45 (q, J = 7.0 Hz, 4H), 1.12 (t, J = 7.0 Hz, 6H); HRMS (ESI): m/z predictive value: C20H19FN2O2 [M+H] + 339.1509, detection value: 339.1510.

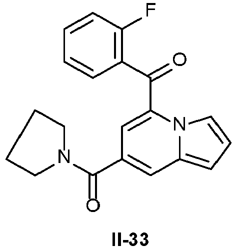

### Embodiment 33 : Preparation of (5-(2-fluorobenzoyl) indolizine-7-yl) (pyrrolidine-1-yl) ketone (II-33)

According to the method of Embodiment 32, a yellow solid (11-33, 81%) is obtained by replacing diethylamine with pyrrolidine (142mg, 2 mmol). 1H NMR (500 MHz, CDC13): δ 8.87 (m, 1H), 7.83 (d, J = 2.0 Hz, 1H), 7.57 (dt, J = 8.0, 1.5 Hz, 1H), 7.52 (m, 2H), 7.33 (1H, dddd, J = 11.5, 8.0, 3.5, 2.0 Hz), 7.29 (1H, d, J = 1.5 Hz), 7.08 (1H, dd, J = 4.5, 2.5 Hz), 6.89 (1H, dd, J = 4.5, 1.0 Hz), 3.57 (m, 4H), 1.82 (m, 4H); HRMS (ESI): m/z predictive value: C20H17FN2O2 [M+H] + 337.1352, detection value: 337.1356.

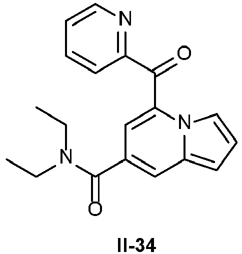

### Embodiment 34: Preparation of N, N-Diethyl-5-pyridylimide-7-formamide (II-34)

According to the method of Embodiment 32, a yellow solid (II-34, 89%) is obtained by replacing compound II-9 with II-17 (1.4g, 5mmol). 1H NMR (500 MHz, CDC13): 9.17 (m, 1H), 8.85 (m, 1H), 8.61 (d, J = 2.0 Hz, 1H), 8.27 (d, J = 2.0 Hz, 1H), 8.09 (dt, J = 8.0, 2.0 Hz, 1H), 7.99 (ddd, J = 8.5, 7.5, 2.0 Hz, 1H), 7.56 (ddd, J = 8.5, 7.0, 1.5 Hz, 1H), 7.13 (dd, J = 4.5, 2.5 Hz, 1H), 7.05 (dd, J = 4.5, 1.5 Hz, 1H), 3.34 (m, 4H), 1.24 (m, 6H); HRMS (ESI): m/z predictive value: C19H19N302 [M+H] + 322.1556, detection value: 322.1557.

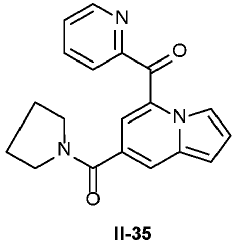

### Embodiment 35 : Preparation of (5-Benzimide-7-yl) (4-methylpiperazin-1-yl) ketone (II-35)

According to the method of Embodiment 32, a yellow solid (II-35, 85%) is obtained by replacing compound II-9 with compound II-17 (1.4g, 5mmol) and replacing diethylamine with pyrrolidine (142mg, 2 mmol).1H NMR (500 MHz, CDC13): 9.11 (m, 1H), 8.76 (m, 1H), 8.57 (m, 1H), 8.23 (d, J = 2.0 Hz, 1H), 8.04 (dt, J = 7.5, 2.0 Hz, 1H), 7.94 (ddd, J = 8.0, 7.0, 1.5 Hz, 1H), 7.52 (ddd, J = 8.0, 7.0, 1.5 Hz, 1H), 7.10 (dd, J = 4.5, 2.5 Hz, 1H), 7.01 (dd, J = 4.5, 1.0 Hz, 1H), 3.49 (m, 4H) 1.74 (m, 4H); HRMS (ESI): m/z predictive value: C19H17N3O2 [M+H] + 320.1399, detection value: 320.1403.

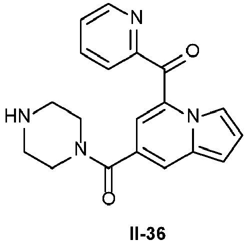

### Embodiment 36 : Preparation of (5-Pyridinyllin-2-yl) (Piperazin-1-yl) Methylketone (II-36)

According to the method of Embodiment 32, a yellow solid (II-36, 73%) is obtained by replacing compound II-9 with compound II-17 and replacing diethylamine with piperazine (172mg, 2 mmol). 1H NMR (500 MHz, CDC13): δ 9.05 (m, 1H), 8.71 (m, 1H), 8.54 (d, J = 1.5 Hz, 1H), 8.18 (d, J = 2.0 Hz, 1H), 7.99 (dt, J = 7.5, 2.0 Hz, 1H), 7.91 (ddd, J = 8.5, 7.0, 1.5 Hz, 1H), 7.48 (ddd, J = 8.5, 7.5, 1.5 Hz, 1H), 7.07 (dd, J = 4.5, 2.5 Hz, 1H), 6.98 (dd, J = 4.5, 1.5 Hz, 1H), 4.13 (s, 1H), 3.54 (m, 4H), 3.14 (m, 4H); HRMS (ESI): m/z predictive value: C19H18N4O2 [M+H] + 335.1508, detection value: 335.1510.

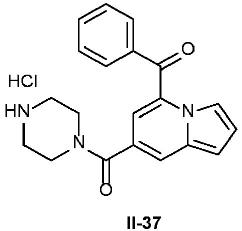

### Embodiment 37: Preparation of (5-Benzimide-7-yl) (piperazin-1-yl) ketone hydrochloride (II-37)

Step e: Dissolving compound II-29 (33mg, 0.1mmol) in ether, adding a hydrochloric acid methanol solution, and reacting at room temperature for 2 hours until the complete reaction of the raw materials is monitored by thin layer chromatography, filtering to obtain a yellow solid (II-37, 98%). 1NMR (500 MHz, CDC13): δ 10.43 (s, 2H), 9.04 (d, J = 1.5 Hz, 1H), 7.94 (m, 3H), 7.73 (t, J = 7.0 Hz, 1H), 7.61 (t, J = 7.0 Hz, 2H), 7.42 (d, J = 2.0 Hz, 1H), 7.13 (dd, J = 4.5, 3.0 Hz, 1H), 6.95 (dd, J = 4.5, 1.5 Hz, 1H), 4.43 (m, 4H), 3.76 (m, 4H); HRMS (ESI): m/z predictive value: C20H20C1N3O2 [M] + 334.1556, detection value: 334.1560.

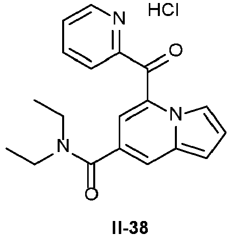

### Embodiment 38: Preparation of N,N-Diethyl-5-pyridylimide-7-formamide hydrochloride (II-38)

According to the method of Embodiment 37, a yellow solid (II-38, 87%) is obtained by replacing compound II-29 with compound II-34 (36mg, 0.1mmol). 1H NMR (500 MHz, CDC13): 11.54 (1H, s), 9.31 (d, J = 2.0 Hz, 1H), 8.95 (m, 2H), 8.46 (d, J = 2.0 Hz, 1H), 8.19 (t, J = 8.0 Hz, 1H), 8.13 (ddd, J = 8.0, 7.0, 2.0 Hz, 1H), 7.72 (ddd, J = 8.0, 7.5, 1.5 Hz, 1H), 7.23 (dd, J = 4.5, 3.0 Hz, 1H), 7.09 (dd, J = 4.5, 1.0 Hz, 1H), 3.54 (m, 4H), 1.37 (m, 6H); HRMS (ESI): m/z predictive value: C19H20C1N302 [M] + 322.1556, detection value: 322.1559.

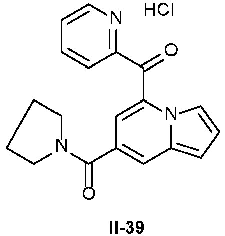

### Embodiment 39: Preparation of (5-methylpyridinimide 7-yl) (pyrrolidine-1-yl) ketone hydrochloride (II-39)

According to the method of Embodiment 37, a yellow solid (II-39, 92%) is obtained by replacing compound II-29 with compound II-35 (32mg, 0.1mmol). 1H NMR (500 MHz, CDC13): 11.27 (1H, s), 9.27 (m, 1H), 8.85 (m, 1H), 8.67 (m, 1H), 8.17 (m, 2H), 8.04 (ddd, J = 8.5, 7.0, 1.5 Hz, 1H), 7.59 (ddd, J = 8.0, 7.0, 1.5 Hz, 1H), 7.19 (dd, J = 4.5, 3.0 Hz, 1H), 7.05 (d, J = 4.0 Hz, 1H), 3.65 (m, 4H) 1.86 (m, 4H); HRMS (ESI): m/z predictive value: C19H18C1N3O2 [M] + 320.1399, detection value: 320.1340.

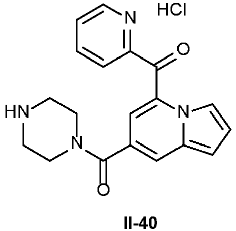

### Embodiment 40: Preparation of (5-pyridinyllindozin-7-yl) (piperazin-1-yl) ketone hydrochloride (II-40)

According to the method of Embodiment 37, a yellow solid (II-40, 91%) is obtained by replacing compound II-29 with compound II-36 (36mg, 0.1mmol). 1H NMR (500 MHz, CDC13): 10.94 (1H, s), 9.16 (m, 1H), 8.87 (m, 1H), 8.62 (d, J = 2.0 Hz, 1H), 8.26 (m, 1H), 8.05 (t, J = 8.0 Hz, 1H), 7.98 (ddd, J = 8.0, 7.5, 1.5 Hz, 1H), 7.54 (ddd, J = 8.0, 7.5, 1.5 Hz, 1H), 7.11 (dd, J = 4.5, 2.5 Hz, 1H), 7.02 (dd, J = 4.5, 1.0 Hz, 1H), 5.34 (s, 1H), 3.78 (m, 4H), 3.47 (m, 4H); HRMS (ESI): m/z predictive value: C19H19C1N4O2 [M] + 335.1508, detection value: 335.1511.

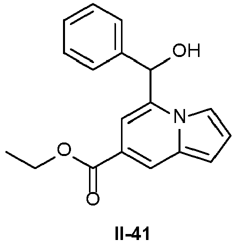

### Embodiment 41 : Preparation of 1- (5- (hydroxy (phenyl) methyl) indolizine-7-yl) propan-1-ketone (II-41)

Step f: Under nitrogen protection, dissolving compound II-1 (293mg, 1mmol) in anhydrous methanol, freezing the reaction solution to 0 °C by ice bath, adding sodium borohydride in batches into the reaction solution, and reacting at 0 ° C for 2 hours, after the complete reaction of the raw materials is monitored by thin-layer chromatography, quenching the reaction solution with saturated ammonium chloride solution under ice bath, and then removing the solvent by reduced pressure distillation, purifying the obtained crude product by silica gel column chromatography to obtain a white solid (II-41, 97%). 1H NMR (500 MHz, CDCl₃): δ 8.24 (d, J = 1.5 Hz, 1H), 7.47 (dt, J = 8.0, 1.5 Hz, 2H), 7.37 (m, 5H), 6.83 (dd, J = 4.0, 3.0 Hz, 1H), 6.79 (dd, J = 4.5, 1.5 Hz, 1H), 6.04 (d, J = 4.0 Hz, 1H), 4.38 (q, J = 7.0 Hz, 2H), 2.70 (d, J = 3.0 Hz, 1H), 1.42 (t, J = 7.0 Hz, 3H); HRMS (ESI): m/z predictive value: C18H17NO3 [M+H] + 296.1287, detection value: 296.1290.

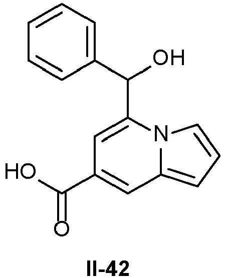

### Embodiment 42: Preparation of 5- (hydroxy (phenyl) methylindolizine-7-carboxylic acid (II-42)

According to the method of Embodiment 41, a white solid (II-42, 94%) is obtained by replacing compound II-1 with compound 11-19 (265mg, 1mmol). 1H NMR (500 MHz, CDCl₃): δ 12.72 (s, 1H), 8.14 (d, J = 1.0 Hz, 1H), 7.63 (s, 1H), 7.42 (d, J = 7.0 Hz, 2H), 7.36 (t, J = 7.0 Hz, 2H), 7.30 (tt, J = 7.0, 1.0 Hz, 1H), 6.86 (dd, J = 3.5, 2.0 Hz, 1H), 6.82 (dd, J = 4.0, 1.0 Hz, 1H), 6.38 (d, J = 4.0 Hz, 1H), 6.01 (d, J = 5.0 Hz, 1H), 3.34 (s, 1H); HRMS (ESI): m/z predictive value: C16H13NO3 [M+H] + 268.0974, detection value: 268.0979.

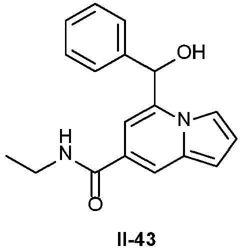

### Embodiment 43 : Preparation of N-ethyl-5-(hydroxy(phenyl)methyl) indolizine-7-formamide (II-43)

According to the method of Embodiment 41, a white solid (II-43, 95%) is obtained by replacing compound II-1 with compound II-21 (293mg, 1mmol) . 1H NMR (500 MHz, CDCl₃): δ 8.01 (d, J = 1.5 Hz, 1H), 7.48 (dt, J = 7.0 Hz, 2H), 7.43 (d, J = 2.0 Hz, 1H), 7.36 (t, J = 7.0 Hz, 2H), 7.30 (t, J = 7.0 Hz, 1H), 7.21 (d, J = 1.5 Hz, 1H), 6.78 (dd, J = 4.0, 3.0 Hz, 1H), 6.71 (dd, J = 4.0, 0.5 Hz, 1H), 6.04 (s, 1H), 4.66 (s, 1H), 3.44 (q, J = 7.0 Hz, 2H), 1.26 (t, J = 7.0 Hz, 3H); HRMS (ESI): m/z predictive value: C18H18N2O2 [M+H] + 295.1447, detection value: 295.1448.

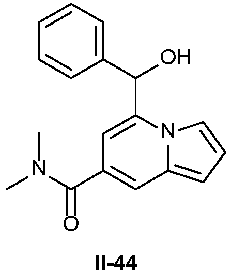

### Embodiment 44 : Preparation of 5-(hydroxy(phenyl)methyl)-N, N-dimethylindolizine-7-formamide (II-44)

According to the method of Embodiment 41, a white solid (II-44, 97%) is obtained by replacing compound II-1 with compound II-22 (293mg, 1mmol). 1H NMR (500 MHz, CDCl3): δ 7.50 (d, J = 1.5 Hz, 1H), 7.41 (dd, J = 7.0, 2.0 Hz, 2H), 7.34 (m, 3H), 7.29 (d, J = 2.0 Hz, 1H), 6.77 (m, 2H), 6.59 (dd, J = 4.0, 1.0 Hz, 1H), 5.97 (s, 1H), 3.36 (s, 1H), 3.06 (s, 6H); HRMS (ESI): m/zpredictive value: C18H18N2O2 [M+H] + 295.1447, detection value: 295.1451.

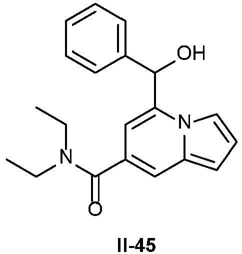

### Embodiment 45 : Preparation of N,N-diethyl-5-(hydroxy(phenyl)methyl) indolizine-7-formamide (II-45)

According to the method of Embodiment 41, a white solid (II-45, 96%) is obtained by replacing compound II-1 with compound II-23 (321mg, 1mmol). 1H NMR (500 MHz, CDCl3): δ 7.44 (d, J = 1.0 Hz, 1H), 7.40 (dd, J = 7.5, 1.5 Hz, 2H), 7.32 (m, 3H), 7.26 (m, 1H), 6.75 (dd, J = 4.0, 3.0 Hz, 1H), 6.71 (s, 1H), 6.56 (dd, J = 4.0, 1.0 Hz, 1H), 5.95 (s, 1H), 3.57 (s, 1H), 3.42 (m, 4H) 1.16 (m, 6H); HRMS (ESI): m/z, predictive value: C20H22N2O2 [M+H] + 323.1760, detection value: 323.1763.

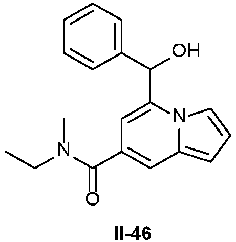

### Embodiment 46: Preparation of N-ethyl-5-(hydroxy(phenyl)methyl)- N-methylindolizine-7-formamide (II-46)

According to the method of Embodiment 41, a white solid (II-46, 97%) is obtained by replacing compound II-1 with compound II-25 (307mg, 1mmol). 1H NMR (500 MHz, CDCl3): δ 7.51 (s, 1H), 7.44 (d, J = 7.0 Hz, 2H), 7.34 (m, 5H), 6.78 (m, 2H), 6.62 (d, J = 4.0 Hz, 1H), 6.00 (s, 1H), 3.46 (m, 2H), 3.05 (s, 3H), 1.20 (m, 3H); HRMS (ESI): m/z predictive value: C19H20N2O2 [M+H] + 309.1603, detection value: 309.1606.

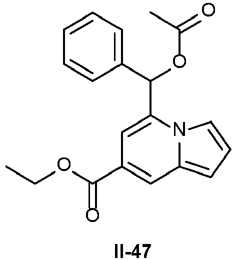

### Embodiment 47: Preparation of 5- (acetoxy (phenyl) methyl) indolizine-7-ethyl carboxylate (II-47)

Step g1: Under nitrogen protection, dissolving compound II-41 (296mg, 1mmol) and triethylamine (122, 1.2mmol) in anhydrous dichloromethane, cooling the reaction solution to 0 °C and dropwisely adding acetyl chloride (94mg, 1.2mmol), after dropwisely adding, reacting at room temperature for 8 hours, after monitoring the complete reaction of the raw materials by thin layer chromatography, dropwisely adding 2 ml saturated ammonium chloride solution into the reaction solution under an ice bath, after dropwisely adding, extracting the reaction solution with dichloromethane (15 ml) and water (15 ml), and extracting the aqueous phase twice with dichloromethane (15 ml), combining the dichloromethane phases, drying with anhydrous sodium sulfate, and removing the solvent by reduced pressure distillation, purifying the obatined crude product by silica gel column chromatography to obtain a light yellow solid (II-47, 82%). 1H NMR (500 MHz, CDCl3): δ 8.28 (d, J = 1.0 Hz, 1H), 7.44 (m, 2H), 7.39 (m, 3H), 7.34 (d, J = 2.5 Hz, 1H), 7.27 (d, J = 1.0 Hz, 1H), 7.19 (s, 1H), 6.86 (dd, J = 4.0, 3.0 Hz, 1H), 6.83 (dd, J = 4.5, 1.0 Hz, 1H), 4.40 (q, J = 7.0 Hz, 2H), 2.24 (s, 3H), 1.43 (t, J = 7.0 Hz, 3H); HRMS (ESI): m/z predictive value: C20H19NO4 [M+H] + 338.1392, detection value: 338.1395.

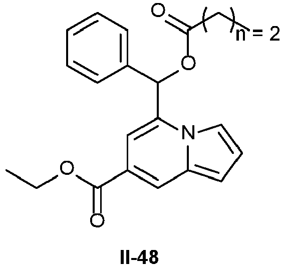

### Embodiment 48: Preparation of Ethyl 5- (butyryloxy) (phenyl) methyl) indolizine-7-carboxylate (II-48)

According to the method of Embodiment 47, a yellow solid (II-48, 77%) is obtained by replacing acetyl chloride with butyryl chloride (127mg, 1.2 mmol). 1H NMR (500 MHz, CDCl3): δ 8.28 (s, 1H), 7.39 (m, 6H), 7.27 (s, 1H), 7.21 (s, 1H), 6.86 (dd, J = 4.0, 2.5 Hz, 1H), 6.83 (dd, J = 3.5, 0.5 Hz, 1H), 4.40 (m, 2H), 2.40 (t, J = 7.5 Hz, 2H), 1.70 (m, 2H) 1.40 (t, J = 7.0 Hz, 3H), 0.97 (t, J = 7.0 Hz, 3H); HRMS (ESI): m/z predictive value: C22H23NO4 [M+H] + 366.1705, detection value: 366.1707.

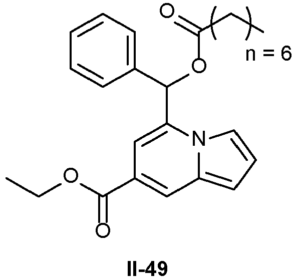

### Embodiment 49 : Preparation of 5- ((octanyloxy) (phenyl) methyl) indolizine-7-ethyl carboxylate (II-49)

According to the method of Embodiment 47, a yellow solid (II-49, 64%) is obtained by replacing acetyl chloride with octyl chloride (194mg, 1.2 mmol). 1H NMR (500 MHz, CDCl3): δ 8.26 (d, J = 1.0 Hz, 1H), 7.43 (m, 2H), 7.36 (m, 3H), 7.31 (d, J = 2.0 Hz, 1H), 7.25 (s, 1H), 7.18 (s, 1H), 6.84 (dd, J = 4.0, 2.5 Hz, 1H), 6.81 (dd, J = 4.0, 1.0 Hz, 1H), 4.37 (t, J = 7.5 Hz, 2H), 2.37 (t, J = 7.5 Hz, 2H), 1.64 (m, 2H) 1.38 (m, 9H), 0.87 (t, J = 7.0 Hz, 3H); HRMS (ESI): m/z predictive value: C25H29NO4 [M+H] + 408.2175, detection value: 408.2177.

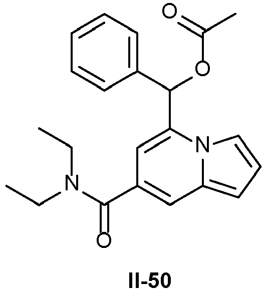

### Embodiment 50: Preparation of (7- (diethylcarbamoyl) azaindole-5-yl) (phenyl) methyl carboxylate (II-50)

According to the method of Embodiment 47, a light yellow solid (II-50, 79%) is obtained by replacing compound II-41 with compound II-45 (322mg, 1 mmol). 1H NMR (500 MHz, CDCl3): δ 7.46 (d, J = 1.5 Hz, 1H), 7.42 (dd, J = 7.0, 2.0 Hz, 2H), 7.34 (m, 3H), 7.21 (d, J = 2.0 Hz, 1H), 6.77 (dd, J = 4.0, 3.0 Hz, 1H), 6.73 (s, 1H), 6.57 (dd, J = 4.0, 1.0 Hz, 1H), 5.96 (s, 1H), 3.41 (m, 4H), 3.07 (s, 3H), 1.19 (m, 6H); HRMS (ESI): m/z predictive value: C22H24N2O3 [M+H] + 365.1865, detection value: 365.1866.

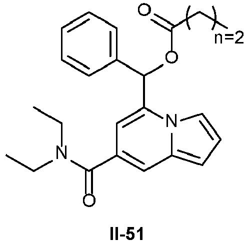

### Embodiment 51: Preparation of (7- (diethylcarbamoyl) azaindole-5-yl) (phenyl) methyl butyrate (11-51)

According to the method of Embodiment 47, a light yellow solid (II-51, 72%) is obtained by replacing compound II-41 with compound II-45 (322mg, 1mmol) and replacing acetyl chloride with butyryl chloride (127mg, 1.2 mmol). 1H NMR (500 MHz, CDCl3): δ 7.43 (m, 1H), 7.39 (d, J = 7.0 Hz, 2H), 7.31 (m, 3H), 7.19 (d, J = 2.0 Hz, 1H), 6.75 (dd, J = 4.0, 2.5 Hz, 1H), 6.71 (s, 1H), 6.54 (dd, J = 4.0, 1.5 Hz, 1H), 5.93 (s, 1H), 3.41 (m, 4H), 2.40 (t, J = 7.5 Hz, 2H), 1.70 (m, 2H), 1.21 (m, 6H), 0.97 (t, J = 7.0 Hz, 3H); HRMS (ESI): m/z predictive value: C24H28N2O3 [M+H] + 393.2178, detection value: 393.2183.

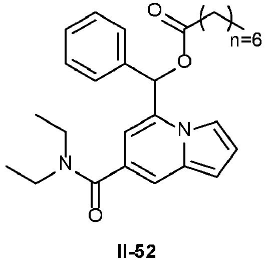

### Embodiment 52: Preparation of (7- (diethylcarbamoyl) azaindole-5-yl) (phenyl) methyl octanoate (II-52)

According to the method of Embodiment 47, a light yellow solid (II-52, 72%) is obtained by replacing compound II-41 with compound II-45 (322mg, 1.0 mmol) and replacing acetyl chloride with octanyl chloride (194mg, 1.2 mmol) (127mg, 1.2 mmol) . 1H NMR (500 MHz, CDCl3): δ 7.42 (d, J = 2.0 Hz, 1H), 7.37 (dd, J = 7.0, 1.5 Hz, 2H), 7.28 (m, 3H), 7.18 (d, J = 2.0 Hz, 1H), 6.72 (dd, J = 4.0, 2.5 Hz, 1H), 6.69 (s, 1H), 6.51 (dd, J = 4.0, 1.0 Hz, 1H), 5.91 (s, 1H), 3.37 (m, 4H), 2.35 (t, J = 7.5 Hz, 2H), 1.31 (m, 15H), 0.92 (t, J = 7.0 Hz, 3H); HRMS (ESI): m/z predictive value: C28H36N2O3 [M+H] + 449.2804, detection value: 449.2805.

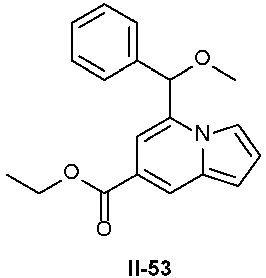

### Embodiment 53 : Preparation of (5-benzimide 7-yl) (4-methylpiperazin-1-yl) ketone (II-53)

Step g2: Under nitrogen protection, dissolving compound II-41 (30mg, 0.1 mmol) in N, N-dimethylformamide (2 mL), cooling the reaction solution to 0 ° C, dropwisely adding sodium hydride (5mg, 0.12 mmol), after dropwisely adding, reacting at 0 ° C for 30 minutes, and then dropwisely adding iodomethane (17mg, 0.12mmol), after dropwisely adding, reacting at room temperature for 8 hours, after monitoring the complete reaction of the raw materials by thin layer chromatography, dropwisely adding 2 ml of saturated ammonium chloride solution into the reaction solution under an ice bath, after dropwisely adding, extracting the reaction solution with 5 mL of ethyl acetate and 5 mL of water, and then extracting the aqueous phase twice with 5 mL of ethyl acetate, combining the ethyl acetate phases, drying with anhydrous sodium sulfate, and removing the solvent by reduced pressure distillation, purifying the obtained crude product by silica gel column chromatography to obtain a light yellow solid (II-53, 85%). 1H NMR (500 MHz, CDCl3): δ 8.14 (m, 1H), 7.36 (t, J = 8.0 Hz, 2H), 7.27 (m, 5H), 6.71 (dd, J = 4.0, 2.5 Hz, 1H), 6.68 (dd, J = 4.5, 1.0 Hz, 1H), 5.68 (s, 1H), 4.35 (q, J = 7.0 Hz, 2H), 3.31 (s, 3H), 1.41 (t, J = 7.0 Hz, 3H); HRMS (ESI): m/z predictive value: C19H19NO3 [M+H] + 310.1443, detection value: 310.1447.

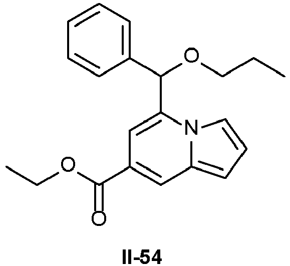

### Embodiment 54: Preparation of 5- (Phenyl (Propyloxy) Methylindolizine-7-ethyl carboxylate

According to the method of Embodiment 53, replacing iodomethane (II-54, 65%) with iodopropane (20mg, 0.12 mmol). δ 8.26 (d, J = 2.0 Hz, 1H), 7.35 (t, J = 8.0 Hz, 2H), 7.29 (d, J = 8.0 Hz, 2H), 7.25 (m, 3H), 6.74 (dd, J = 4.0, 2.5 Hz, 1H), 6.63 (dd, J = 4.5, 1.5 Hz, 1H), 5.72 (s, 1H), 4.37 (q, J = 7.0 Hz, 2H), 3.2 (t, J = 7.0 Hz, 2H), 1.52 (m, 2H), 1.41 (t, J = 7.0 Hz, 3H), 0.94 (t, J = 7.0 Hz, 3H); HRMS (ESI): m/z predictive value: C21H23NO3 [M+H] + 338.1756, detection value: 338.1758.

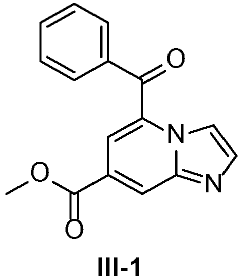

### Embodiment 55: Preparation of 5-benzimidazolo[1,2-a]pyridine-7-methyl carboxylate (III-1)

Step h: Under nitrogen protection, dissolving imidazole (680mg, 10mmol) and potassium carbonate (1.7mg, 12mmol) in anhydrous acetonitrile (30ml), then adding 2-bromo-1-phenylethane-1-one (2.4g, 12mmol) in batches, after complete addition, reacting overnight at 60 °C, after monitoring the complete reaction of the raw materials by thin-layer chromatography, cooling to room temperature, then filtering to remove potassium carbonate, and reduced pressure distilling the filtrate to remove the solvent, purifying the obtained crude product by silica gel column chromatography to obtain a white solid product, which is directly used for the next step of the reaction;

Step i: Under nitrogen protection, dissolving the previous product 2-(1H-imidazol-1-yl)-1-phenylethane-1-ketone (930mg, 5mmol) in anhydrous dichloromethane (30ml), then adding phosphorus oxychloride (912mg, 6mmol), and freezing the reaction solution to 0 °C in ice bath, slowly adding N,N-dimethylformamide (438mg, 6mmol) into the reaction solution, and reacting at 0 °C for 2 hours, after monitoring the complete reaction of the raw materials by thin layer chromatography, extracting the reaction solution with 50 mL of ethyl acetate and 50 mL of water, and extracting the aqueous phase twice with 50 mL of ethyl acetate, and combining the ethyl acetate phases, drying with anhydrous sodium sulfate, and removing the solvent by reduced pressure distillation, purifying the crude obtained product by silica gel column chromatography to obtain a white solid product;

Step j: Under nitrogen protection, dissolving the product 1- (2-oxo-2-phenylethyl) -1H-imidazo-2-aminoformaldehyde obtained from the previous step (428mg, 2mmol), methyl propiolate (202mg, 2.4mmol), and potassium carbonate (331mg, 2.4mmol) in anhydrous N, N-dimethylformamide (6 milliliters), and reacting overnight at 80 °C, after monitoring the complete reaction of the raw materials by thin layer chromatography, cooling to room temperature, extracting the reaction solutionwith 15 mL of ethyl acetate and 15 mL of water, and extracting the aqueous phase twice with 15 mL of ethyl acetate, combining the organic phases, drying with anhydrous sodium sulfate, and removing the solvent by reduced pressure distillation, purifying the obtained crude product by silica gel column chromatography to obtain a yellow solid (III-1, 74%). 1H NMR (500 MHz, CDCl3): δ 8.91 (s, 1H), 8.61 (d, J = 1.0 Hz, 1H), 7.99 (d, J = 1.0 Hz, 1H), 7.95 (d, J = 1.5 Hz, 1H), 7.83 (dt, J = 7.0, 1.5 Hz, 2H), 7.68 (tt, J = 7.5, 1.0 Hz, 1H), 7.56 (t, J = 7.5 Hz, 2H), 3.95 (s, 3H); HRMS (ESI): m/z predictive value: C16H12N2O3 [M+H] + 282.0926, detection value: 282.0929.

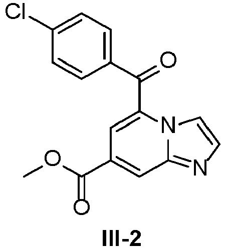

### Embodiment 56: Preparation of 5- (4-chlorobenzoyl) imidazolo [1,2-a] pyridine-7-methyl carboxylate (III-2)

According to the method of Embodiment 55, a yellow solid (III-2, 67%) is obtained by replacing 2-bromo-1-phenylethane-1-ketone with 2-bromo-1- (4-chlorophenyl) ethane-1-ketone (2.8g, 12mmol). 1H NMR (500 MHz, CDCl3): δ 8.93 (d, J = 1.5 Hz, 1H), 8.64 (d, J = 1.0 Hz, 1H), 8.01 (d, J = 1.0 Hz, 1H), 7.97 (d, J = 1.5 Hz, 1H), 7.86 (d, J = 7.0 Hz, 2H), 7.57 (d, J = 7.5 Hz, 2H), 3.92 (3H, s); HRMS (ESI): m/z predictive value: C16H11ClN2O3 [M+H] + 315.0536, detection value: 315.0538.

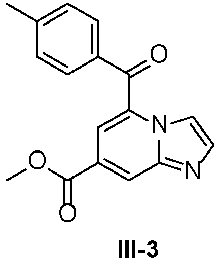

### Embodiment 57 : Preparation of 5- (4-methylbenzoyl) imidazolo [1,2-a] pyridine-7-methyl carboxylate (III-3)

According to the method of Embodiment 55, a yellow solid (III-3, 82%) is obtained by replacing 2-bromo-1-phenylethane-1-ketone with 2-bromo-1-(p-tolyl) ethane-1-ketone (2.5g, 12mmol). 1H NMR (500 MHz, CDCl3): δ 8.87 (m, 1H), 8.54 (d, J = 1.5 Hz, 1H), 7.94 (d, J = 1.5 Hz, 1H), 7.91 (d, J = 1.5 Hz, 1H), 7.79 (d, J = 7.0 Hz, 2H), 7.51 (d, J = 7.0 Hz, 2H), 3.96 (3H, s); HRMS (ESI): m/z predictive value: C17H14N2O3 [M+H] + 295.1083, detection value: 295.1088.

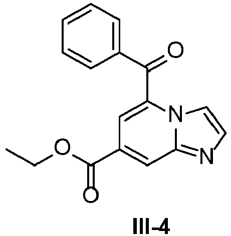

### Embodiment 58: Preparation of 5-benzimidazolo[1,2-a]pyridine-7-ethyl carboxylate (III-4)

According to the method of Embodiment 55, a yellow solid (III-4, 58%) is obtained by replacing methyl propiolate with ethyl propiolate (235 mg, 2.4 mmol) . 1H NMR (500 MHz, CDCl3): δ 8.89 (s, 1H), 8.57 (d, J = 1.0 Hz, 1H), 7.96 (d, J = 1.0 Hz, 1H), 7.94 (d, J = 1.5 Hz, 1H), 7.80 (dt, J = 7.0, 1.5 Hz, 2H), 7.66 (tt, J = 7.0, 1.5 Hz, 1H), 7.54 (t, J = 7.0 Hz, 2H), 4.17 (q, J = 7.0 Hz, 2H), 1.41 (t, J = 7.0 Hz, 3H); HRMS (ESI): m/z predictive value: C17H14N2O3 [M+H] + 295.1083, detection value: 295.1085.

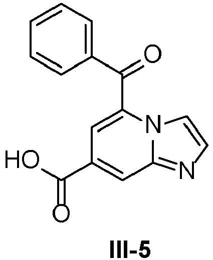

### Embodiment 59 : Preparation of 5-benzimide azo [1,2-a] pyridine-7-carboxylic acid (III-5)

Step k: Dissolving compound III-4 (588mg, 2mmol) in 6 mL of ethanol, adding 2N sodium hydroxide solution (2 mL), and stirring the reaction solution overnight at 80 ° C, after monitoring the complete reaction of the raw materials by thin layer chromatography, removing the ethanol by reduced pressure distillation, adjusting the remaining solution to acidity with hydrochloric acid solution, and a yellow solid precipitating, filtering, washing the solid with water, and drying to obtain a yellow solid (III-5, 94%).1H NMR (500 MHz, CDCl3): δ 12.27 (1H, s), 8.87 (s, 1H), 8.54 (s, 1H), 7.93 (d, J = 1.5 Hz, 1H), 7.91 (d, J = 1.5 Hz, 1H), 7.78 (dt, J = 7.0, 1.0 Hz, 2H), 7.63 (tt, J = 7.0, 1.0 Hz, 1H), 7.52 (t, J = 7.0 Hz, 2H); HRMS (ESI): m/z predictive value: C15H10N2O3 [M+H] + 267.0770, detection value: 267.0771.

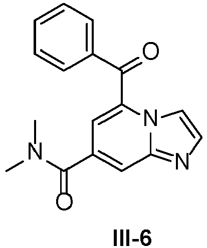

### Embodiment 60 : Preparation of 5-Benzoyl-N,N-dimethylimidazole[1,2-a] pyridine-7-formamide (III-6)

Step 1: Dissolving compound III-5 (53mg, 0.2mmol), 1-hydroxybenzotriazole (13mg, 0.06mmol), 1-ethyl - (3-dimethylaminopropyl) carbodiimide hydrochloride (46mg, 0.24mmol) in 2ml anhydrous tetrahydrofuran, adding N,N-diisopropylethylamine (34mg, 0.26mmol) at room temperature, and reacting for 8 hours at room temperature until the complete reaction of III-5 is monitored by thin layer chromatography, then adding dimethylamine (11mg, 0.24mmol) and reacting for 2 hours at room temperature, after monitoring the complete reaction of the raw materials by thin layer chromatography, reduced pressure distilling the reaction solution to remove the solvent, and purifying the obtainted crude product by silica gel column chromatography to obtain a yellow solid (III-6, 86%). 1H NMR (500 MHz, CDCl3): δ 8.87 (d, J = 2.0 Hz, 1H), 8.55 (d, J = 1.5 Hz, 1H), 7.92 (d, J = 1.5 Hz, 1H), 7.87 (d, J = 1.5 Hz, 1H), 7.73 (dt, J = 7.5, 1.5 Hz, 2H), 7.67 (tt, J = 7.5, 1.5 Hz, 1H), 7.50 (t, J = 7.0 Hz, 2H), 3.27 (s, 6H); HRMS (ESI): m/z predictive value: C17H15N3O2 [M+H] + 294.1243, detection value: 294.1245.

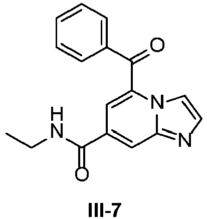

### Embodiment 61: Preparation of 5-benzoyl-N-ethylimidazolo[1,2-a] pyridine-7-formamide (III-7)

According to the method of Embodiment 60, a yellow solid (III-7, 88%) is obtained by replacing dimethylamine with ethylamine (11mg, 0.24mmol) . 1H NMR (500 MHz, CDCl3): δ 8.85 (m, 1H), 8.56 (d, J = 1.5 Hz, 1H), 7.94 (d, J = 1.0 Hz, 1H), 7.86 (d, J = 1.0 Hz, 1H), 7.74 (dt, J = 7.5, 1.5 Hz, 2H), 7.65 (tt, J = 7.0, 1.5 Hz, 1H), 7.50 (t, J = 7.5 Hz, 2H), 5.85 (m, 1H), 3.47 (m, 2H), 1.24 (t, J = 7.0 Hz, 3H); HRMS (ESI): m/z predictive value: C17H15N3O2 [M+H] + 294.1243, detection value: 294.1247.

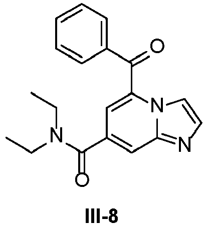

### Embodiment 62: Preparation of 5-Benzoyl-N,N-diethylimidazole[1,2-a] pyridine-7-formamide (III-8)

According to the method of Embodiment 60, a yellow solid (III-8, 79%) is obtained by replacing dimethylamine with diethylamine (18mg, 0.24mmol). 1H NMR (500 MHz, CDCl3): δ 8.86 (m, 1H), 8.54 (d, J = 1.5 Hz, 1H), 7.91 (d, J = 1.5 Hz, 1H), 7.89 (d, J = 1.5 Hz, 1H), 7.74 (dt, J = 7.0, 1.0 Hz, 2H), 7.65 (tt, J = 7.0, 1.0 Hz, 1H), 7.48 (t, J = 7.0 Hz, 2H), 3.56 (m, 4H), 1.14 (m, 6H); HRMS (ESI): m/z predictive value: C19H19N3O2 [M+H] + 322.1556, detection value: 322.1557.

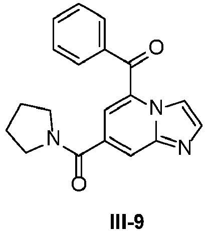

### Embodiment 63 : Preparation of (5-benzoimidazolo[1,2-a]pyridine-7-yl) (pyrrolidine-1-yl) ketone (III-9)

According to the method of Embodiment 60, a yellow solid (III-9, 73%) is obtained by replacing dimethylamine with pyrrolidine (17mg, 0.24mmol). 1H NMR (500 MHz, CDCl3): δ 8.86 (d, J = 1.5 Hz, 1H), 8.56 (d, J = 1.5 Hz, 1H), 7.93 (d, J = 1.5 Hz, 1H), 7.87 (d, J = 1.5 Hz, 1H), 7.72 (dt, J = 7.0, 1.0 Hz, 2H), 7.63 (tt, J = 7.0, 1.0 Hz, 1H), 7.50 (t, J = 7.0 Hz, 2H), 3.63 (m, 4H), 1.43 (m, 4H); HRMS (ESI): m/z predictive: value C19H17N3O2 [M+H] + 320.1399, detection value: 320.1402.

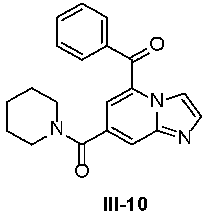

### Embodiment 64: Preparation of (5-benzimidazole[1,2-a] pyridine-7-yl) (piperidin-1-yl) ketone (III-10)

According to the method of Embodiment 60, a yellow solid (III-10, 77%) is obtained by replacing dimethylamine with piperidine (20mg, 0.24mmol) . 1H NMR (500 MHz, CDCl3): δ 8.89 (m, 1H), 8.55 (d, J = 1.0 Hz, 1H), 7.93 (d, J = 1.5 Hz, 1H), 7.90 (d, J = 1.5 Hz, 1H), 7.72 (dt, J = 7.0, 1.0 Hz, 2H), 7.67 (tt, J = 7.0, 1.0 Hz, 1H), 7.50 (t, J = 7.0 Hz, 2H), 3.74 (m, 4H), 1.95 (m, 4H), 1.47 (m, 2H); HRMS (ESI): m/z predictive value: C20H19N3O2 [M+H] + 334.1556, detection value: 334.1558.

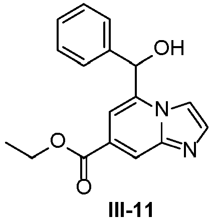

### Embodiment 65: Preparation of 5- (hydroxy (phenyl) methylimidazolo [1,2-a] pyridine-7-ethyl carboxylate (III-11)

Step m: Under nitrogen protection, dissolving compound III-4 (294 mg, 1 mmol) in anhydrous methanol (4 mL), and freezing the reaction solution to 0 °C in an ice bath, adding sodium borohydride (152 mg, 4 mmol) into the reaction solution in batches, and reacting out at 0 °C for 2 hours, after monitoring the complete reaction of the raw materials by thin layer chromatography, quenching the reaction solution with 5 mL of saturated ammonium chloride solution under an ice bath, and removing the solvent by reduced pressure distillation, purifying the obtained crude product by silica gel column chromatography to obtain a white solid (III-11, 93%). 1H NMR (500 MHz, CDCl3): δ 8.37 (m, 1H), 7.51 (d, J = 1.5 Hz, 1H), 7.28 (m, 2H), 7.21 (dt, J = 7.0, 1.5 Hz, 2H), 7.14 (tt, J = 7.0, 1.5 Hz, 1H), 7.03 (t, J = 7.0 Hz, 2H), 5.83 (s, 1H), 4.52 (s, 1H), 4.15 (q, J = 7.0 Hz, 2H), 1.40 (t, J = 7.0 Hz, 3H); HRMS (ESI): m/z predictive value: C17H16N2O3 [M+H]+ 297.1239, detection value: 297.1243.

### Embodiment 66: Biological Evaluation

The fused heterocyclic compound of the present invention has an inhibitory effect on the TRPM2 channel, and its pharmacological activity is measured using whole cell patch clamp technology, the corresponding steps are as follows:
Step 1: Cell preparation
   Incubating HEK293 cells stably expressing human TRPM2 channels in DMEM/F-12 medium containing 10% bovine serum, 50 units/mL of penicillin and 50 mg/mL of streptomycin under the condition of 5% CO2 and 37 ° C.
Step 2: Electrophysiological testing

The cells before testing are stored in the extracellular fluid (147 mM NaCl, 2 mM KCl, 1 mM MgCl2, 2 mM CaCl2, 10 mM HEPES, 13 mM glucose, pH 7.4). The electrode is filled with the electrode internal solution (147 mM NaCl, 0.05 mM EGTA, 1 mM MgCl2, 10 mM HEPES, 0.5 mM ADPR, pH=7.3), and the resistance is maintained at 3-5 M Ω.

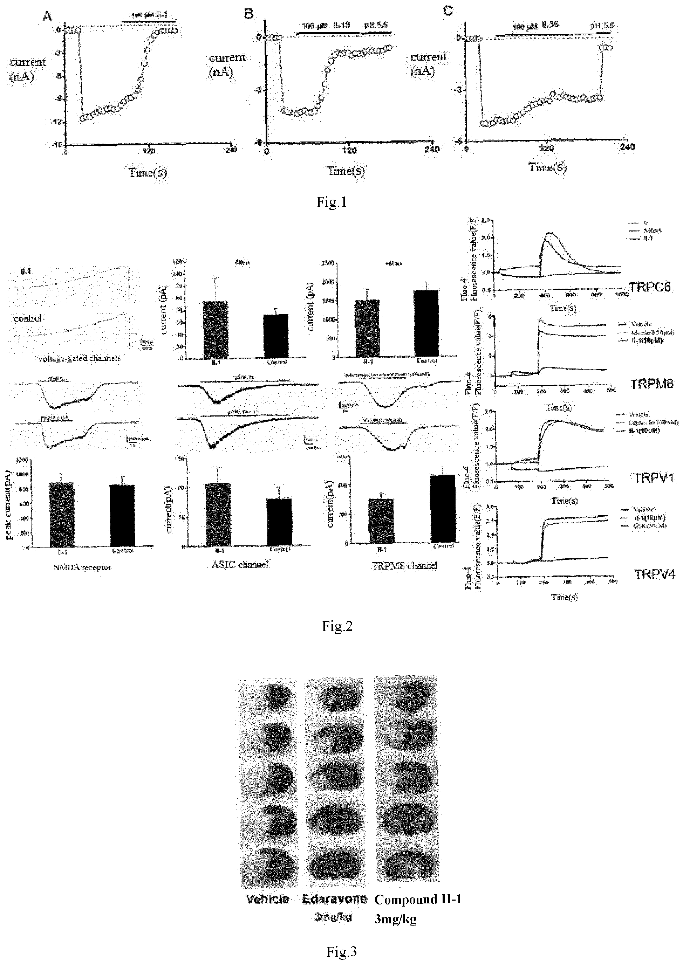

Administering ADPR into cells through electrodes, and detecting the ADPR concentration used to activate TRPM2 as 500 µM, when detecting a stable current, perfusing the corresponding concentration of fused heterocyclic compound II-1 into the extracellular fluid for at least 60 seconds. record the change in current magnitude using voltage ramp mode, changing the voltage from -100 mV to+100 mV within 500 ms, with a normal clamp voltage of 0 mV and a TRPM every 5 seconds; finally, replacing the extracellular fluid with a solution with pH=5.0 to block the TRPM2 current, the electrophysiological current test results obtained are shown in Table 1, and the electrophysiological current graph obtained is shown in Figure 1.

Similarly, administering corresponding concentrations of other fused heterocyclic compounds (II-2~II-54, III-1~III-11) into the extracellular fluid, taking electrophysiological tests using the same method, the results are shown in Table 1. The electrophysiological representative current graphs of some fused heterocyclic compounds (II-9, II-19, and II-36) inhibiting TRPM2 channel current are shown in Figure 1.

**Table 1 IC50 data of some fused heterocyclic compounds on TRPM2 inhibition**

| 20-50µM . +; 10-20µM: ++; 1-10µM: +++. | | | | | | | |
|---|---|---|---|---|---|---|---|
| Embodiment | activity | Embodiment | activity | Embodiment | activity | Embodiment | activity |
| **11-1** | +++ | **II-2** | +++ | **II-3** | ++ | **11-4** | ++ |
| **II-5** | ++ | **11-6** | +++ | **II-7** | +++ | **II-8** | +++ |
| **11-9** | +++ | **II-10** | +++ | **11-11** | +++ | **11-12** | ++ |
| **11-13** | +++ | **11-14** | +++ | **11-15** | ++ | **11-16** | +++ |
| **11-17** | +++ | **11-18** | +++ | **11-19** | ++ | **11-20** | ++ |
| **11-21** | ++ | **11-22** | ++ | **11-23** | +++ | **11-24** | +++ |
| **11-25** | +++ | **11-26** | +++ | **11-27** | ++ | **11-28** | ++ |
| **11-29** | + | **11-30** | + | **11-31** | ++ | **11-32** | ++ |
| **11-33** | ++ | **11-34** | +++ | **11-35** | ++ | **11-36** | + |
| **11-37** | ++ | **11-38** | + | **11-39** | ++ | **11-40** | ++ |
| **11-41** | ++ | **11-42** | ++ | **11-43** | ++ | **11-44** | ++ |
| **11-45** | ++ | **11-46** | ++ | **11-47** | ++ | **11-48** | ++ |
| **11-49** | + | **11-50** | ++ | **11-51** | + | **11-52** | + |
| **11-53** | +++ | **11-54** | ++ | **111-1** | +++ | **III-2** | ++ |
| **III-3** | ++ | **III-4** | ++ | **III-5** | ++ | **III-6** | ++ |
| **III-7** | +++ | **III-8** | ++ | **III-9** | ++ | **III-10** | ++ |
| **III-11** | + | | | | | | |

### Embodiment 67: TRPM2 channel specificity test experiment

According to the method of Example 66, testing the inhibitory effect of the fused heterocyclic compound II-1 of the present invention on different channels to determine the specificity of the compound acting on the TRPM2 channel.

After applying voltage clamp, agonist NMDA, acidic pH, menthol, M085, GSK, and capsaicin to HEK293T cells stably expressing voltage gated channels, NMDA receptors, ASIC channels, TRPM8 channels, TRPC6 channels, TRPV4 channels, and TRPV1 channels, respectively, clamping the voltage inside and outside the cell membrane to -100 mV using patch clamp technology. When the electric potential inside and outside the cell membrane changes, the machine will input compensation current, which can measure the magnitude of the inward current of the cell and reflecting the degree of channel activation through the magnitude of the current. After adding the compound, determining whether the compound has inhibitory effects on the activation of the aforementioned channels by the change of the current magnitude, the measured results are shown in Figure 2.

The test results indicate that the fused heterocyclic compound of the present invention has specificity on inhibiting TRPM2 current, and has no inhibitory effect on voltage gated channels, NMDA receptors, acid channels, TRPM8 channels, TRPC6 channels, TRPV1 channels, TRPV4 channels, etc.

Embodiment 68: Study of the protective activity of the fused ring heterocyclic compound II-1 of the present invention on ischemia/reperfusion injury

### 1) Preparation of a mouse transient focal cerebral ischemia middle cerebral artery embolism (tMCAO) model

After isoflurane inhalation gas anesthesia of 22-25 g male C57BL/6 mice, the fiber-optic probes of the laser Doppler flowmeter were fixed to the animal's skull with glue for monitoring the cerebral blood flow of the middle cerebral artery (MCA) supplying cortex. The mice were fixed in the supine position, and the middle of the neck was dehaired and routinely sterilized with 75% alcohol, an approximately 1.5 cm incision was made in the middle of the neck, and the skin of the neck was incised, and the muscle and fascia were separated along the inner edge of the sternocleidomastoid muscle; the right carotid sheath was bluntly separated to expose the right common carotid artery (CCA), external carotid artery (ECA) and internal carotid artery (ICA); after satisfactory exposure of the surgical area, the CCA and ICA were temporarily clipped with arterial clips, a small opening was cut at the proximal end of the ECA with microsurgical scissors, and a nylon monofilament suture was inserted along the ECA from the small opening, gently pushed into the intracranial area in the direction of the ICA for about 11.5±0.5 mm, and blocking the beginning of the middle cerebral artery. Only animals whose blood flow values decreased to 20% of the basic value were selected according to the blood flow data monitored by the flowmeter. After 90 minutes of ischemia, the monofilament suture was gently withdrawn from the mouse, the arterial stump is tied tightly, the surgical wound was inspected, the bleeding was thoroughly stopped, a small amount of penicillin powder was sprinkled locally to prevent infection, and the subcutaneous tissues and skin were sutured layer by layer, thus completing the ischemia-reperfusion injury model. A heating plate was used to maintain the body temperature of the animals during surgery. After completion of the surgery, the sutured mouse was placed in a heat preservation and humidification box until the mouse recovered activity. Animals in the sham-operated group were subjected to the same surgical procedure but without middle artery occlusion.

### 2) Animal grouping and dosing regimen

Clean-grade male C57BL/6 mice were randomly divided into 3 groups, namely, the sham-operated group, the group of the positive control drug Edaravone ( 3 mg/kg) and the 3 mg/kg dose group of compound II-1. The mice were subjected to tail vein injection of Edaravone and II-1 respectively 3h after modeling and reperfusion; the sham-operated group was injected with an equal volume of saline, and both II-1 and Edaravone were dissolved in 0.9% saline solution. Neurological function scores and TTC staining of mice were performed 24h after ischemia-reperfusion, and the results are shown in Figures 3, 4 and 5.

### (3) Measurement of cerebral infarction volume

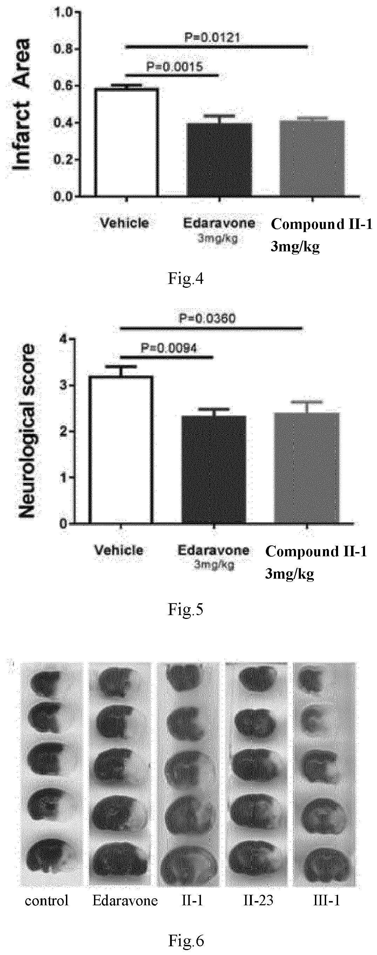

The mice were decapitated 24h after ischemia-reperfusion, the coronal planes of the brains were cut into brain slices of about 2mm thickness uniformly, the brain slices were placed in 0.25% 2,3,5-triphenyltetrazolium chloride (TTC) solution, incubated at 37°C Celsius for 30 minutes away from light, stained, and fixed in 4% paraformaldehyde neutral buffer (pH 7.4) overnight. The processed brain slices were photographed using a macro camera, and Image J image analysis software was used to calculate and count the cerebral infarct volume, and the measured results are shown in Fig. 4. The formula for calculating the percentage of infarct volume was (sum of the left uninfarcted area - sum of the right uninfarcted area)/sum of the left uninfarcted area×l00%.

### 4) Neurological function test

According to the five-level, four-point standard of score, the mortality and neuromotor dysfunction scores of each group of mice were performed, and the results are shown in Fig. 5. The standard of the neurological function score is as follows: 0 point: no symptoms of neurological deficits; 1 point: the forelimb on the side opposite to the cerebral ischemia could not be fully extended when the tail was lifted; 2 points: the animal turned circle to the side opposite to the cerebral ischemia; 3 points: the animal fell to the side opposite to the cerebral ischemia when crawled; 4 points: the animal was unable to walk independently, and lost consciousness.

### 5) Evaluation of activity results

The fused heterocyclic compounds II-1 of the present invention can significantly reduce the volume of cerebral infarction caused by tMCAO in mice and significantly reduce the scores of neuromotor dysfunction caused by tMCAO, and it has similar pharmacological activity with the positive control drug Edaravone at equal concentration.

Embodiment 69: Preventive and therapeutic effects of the fused heterocyclic compounds II-1, II-23 and III-1 of the present invention on cerebral stroke

### 1) Preparation of a mouse transient focal cerebral ischemia middle cerebral artery embolism (tMCAO) model

After isoflurane inhalation gas anesthesia of 22-25 g male C57BL/6 mice, the fiber-optic probes of a laser Doppler flowmeter were fixed to the skull of the mice with glue for monitoring the cerebral blood flow of the middle cerebral artery (MCA) supplying cortex. The mice were fixed in the supine position, and the middle of the neck was dehaired and routinely sterilized with 75% alcohol. An approximately 1.5 cm incision was made in the middle of the neck, and the skin of the neck was incised, and the muscle and fascia were separated along the inner edge of the sternocleidomastoid muscle; the right carotid artery sheath was bluntly separated to expose the right common carotid artery (CCA), external carotid artery (ECA), and internal carotid artery (ICA); after the surgical area was exposed satisfactorily, the CCA and ICA were temporarily clipped with arterial clips, and a small incision was made at the proximal end of the ECA with microsurgical scissors, and a nylon monofilament suture was inserted from the small incision along the ECA, and gently pushed into the intracranial area in the direction of the ICA for about 11.5 ± 0.5 mm, blocking the beginning of the middle cerebral artery. Only the animals whose blood flow values decreased to 20% of the basic value were selected according to the blood flow data monitored by the flowmeter. After 90 minutes of ischemia, the monofilament sutures were gently withdrawn out, the arterial stumps were tied tightly, the surgical wounds were inspected, the bleeding was thoroughly stopped, a small amount of penicillin powder was sprinkled locally to prevent infection, and the subcutaneous tissues and the skin were sutured layer by layer, thus completing the ischemia reperfusion injury model. A heating plate was used to maintain the body temperature of the animals during surgery. After completion of the surgery, the sutured mice were placed in a heat preservation and humidification box until the mice recovered activities. Animals in the sham-operated group were subjected to the same surgical procedure but without middle artery occlusion.

### 2) Animal grouping and dosing regimen

Clean-grade male C57BL/6 mice were randomly divided into five groups, namely, the sham-operated group, the group of the positive control drug Edaravone( 3 mg/kg), and the 3 mg/kg dose group of compounds II-1, II-23 and III-1.

The mice were subjected to tail vein injections of Edaravone, II-1, II-23, and III-1 respectively 3h after modeling and reperfusion; the sham-operated group was injected with an equal volume of saline, and II-1, II-23, III-1, and Edaravone were dissolved in 0.9% saline solution. Neurological function scores and TTC staining of mice were performed 24 h after ischemia-reperfusion, and the results are shown in Figures 6, 7 and 8.

### (3) Measurement of cerebral infarction volume

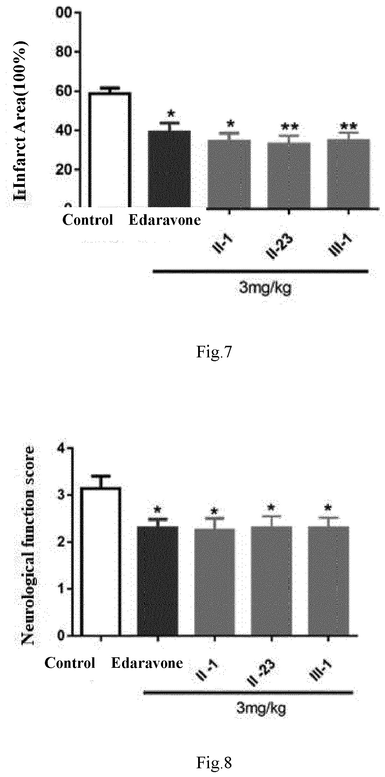

The mice were decapitated 24h after ischemia-reperfusion, the coronal planes of the brains were cut into brain slices of about 2mm thickness uniformly. The brain slices were placed in 0.25% 2,3,5-triphenyltetrazolium chloride (TTC) solution, incubated at 37°C Celsius for 30 minutes away from light, stained, and fixed in 4% paraformaldehyde neutral buffer (pH 7.4) overnight. The processed brain slices were photographed using a macro camera, and Image J image analysis software was used to calculate and count the cerebral infarct volume, and the measured results are shown in Fig. 7. The formula for calculating the percentage of infarct volume was (sum of the left uninfarcted area - sum of the right uninfarcted area)/sum of the left uninfarcted area×l00%.

### 4) Neurological function test

According to the five-level, four-point standard of score, the mortality and neuromotor dysfunction scores of each group of mice were performed, and the results are shown in Fig. 8. The standard of the neurological function score is as follows: 0 point: no symptoms of neurological deficits; 1 point: the forelimb on the side opposite to the cerebral ischemia could not be fully extended when the tail was lifted; 2 points: the animal circled to the side opposite to the cerebral ischemia; 3 points: the animal fell to the side opposite to the cerebral ischemia when crawled; 4 points: the animal was unable to walk independently, and lost consciousness.

### 5) Evaluation of activity results

The fused heterocyclic compounds II-1, II-23 and III-1 of the present invention all significantly reduced the area of cerebral infarction caused by tMCAO in mice and significantly reduced the neuromotor dysfunction scores caused by tMCAO, and had similar pharmacological activities as the positive control drug Edaravone at equal concentrations.

Embodiment 70: Protective effect of knockout of TRPM2 channels on ischemia/reperfusion injury

### 1) Acute hippocampal brain slices

Mice with shiny fur at 3~4 weeks (21 to 28 days after birth) were taken as experimental mice. Prior to the experiment, 200 mL of the slice solution was taken into a beaker, supplied with a 95% O2/5% CO2 gas mixture for 15 minutes, and placed in a -80°C refrigerator to freeze for 25 minutes. While freezing the slice solution, an incubator (homemade, 250 mL beaker) was placed in a water bath at 34°C, and about 180 mL of ACSF was added to the incubator, a gas mixture was continuously supplied. The frozen slice solution was taken out and continuously supplied with oxygen. At this time, the mice were anesthetized with ether, decapitated, and the brains were rapidly taken out on ice (less than 1 min), and the brains were immediately placed in the frozen slice solution and frozen for 5 mins. The brains were removed from the slice solution and the right and left hippocampi were separated on a glass dish (a glass dish placed on ice). The separated hippocampi were placed on a 4% agarose block, and after carefully blotting the surrounding water with filter paper, the agarose block with the hippocampi was fixed on the platform of a vibrating slicer (Leica 1200S) with 502 glue, and the frozen slice solution was immediately poured into the slice trough. When slicing, the direction of the blade edge was perpendicular to the long axis of the hippocampus, and the thickness of the slice was 300 µm. The sliced hippocampal brain slices were transferred with a dropper to the incubation tank, and incubated at 34°C for 30 min, and then the incubation tank was transferred to room temperature. The brain slices were incubated at room temperature for at least 45 min before proceeding to the next step of the experiment, and a 95% O2/5% CO2 gas mixture was continuously supplied to the slice solution and ACSF to maintain the pH of the liquid and supply oxygen to the brain slices.

### 2) Ischemia and hypoxia experiments on brain slices

Incubated acute hippocampal brain slices were randomly divided into Sham and OGD groups. Sham group brain slices and OGD group brain slices were taken out by a dropper and placed in a normal ACSF and a OGD-ACSF for 60 min (34°C), respectively. After the OGD treatment was completed, they were placed back into the normal ACSF for 30 min (34°C). Brain slices were incubated with 5 µg/mL propidium iodide (PI) and 20 µM Newport green (Invetrogen) + ACSF for 10 min at room temperature, the volume of ACSF is 2 ml.Brain slices were incubated in a dye incubator, while ensuring oxygen supply of ACSF and minimizing the effect of mechanical vibration on cellular activity, PI was used to label the damaged cells, and Newport Green was used to detect the amount of zinc ions in neurons. Brain slices incubated with the dye were taken out and placed in a 200 mL ACSF incubator to remove the excess dye. Finally, the brain slices were placed in a recording tank and photographed using a confocal microscope Olympus FV 1000 60X aqueous microscope, and the recording tank was continuously perfused with ACSF. ACSF was required a 95% O2/5% CO2 gas mixture to be supplied 30 mins in advance, and oxygen was also supplied all the time during the experiment; a 95% O2/5% CO2 gas mixture was supplied 30 min before the OGD-ACSF experiment to exclude O2 from the fluid, and N2 was also continuously vented during the OGD treatment.

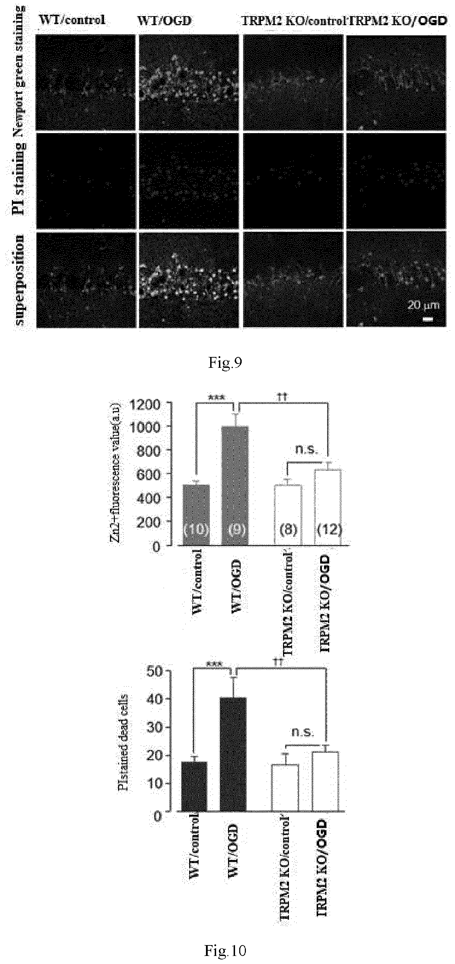

Hippocampal brain slices were obtained from wild-type mice and TRPM2 knockout mice, respectively, and were modeled using ex vivo oxygen-glucose deprivation reperfusion (OGD) to simulate in vivo cerebral ischemic injury, after the OGD was performed for 30 minutes, the hippocampal CA1 neuronal deaths in the mice brain slices were monitored by PI staining, and at the same time the amount of zinc ions in neuronal cells was monitored by Newport Green staining. The test results are shown in Fig. 9. According to the test results in Fig. 9, after the OGD was performed for 30 minutes, a significant increase in hippocampal CA1 neuronal death in brain slices of wild-type mice was observed using PI staining, and the results of zinc ion staining also showed that a large amount of intracellular zinc ions were accumulated in neurons. In contrast, in hippocampal brain slices of TRPM2 knockout mice, we found that both the OGD-triggered zinc ion accumulation and the increased PI staining were significantly suppressed, indicating that TRPM2 deficiency can play a cerebroprotective role by reducing neuronal zinc ion accumulation.

The above experiments indicate that the fused heterocyclic compounds of the present invention have an inhibitory effect on TRPM2 channels, and the inhibition of TRPM2 channels can play a cerebroprotective role by reducing neuronal zinc ion accumulation, and thus the fused heterocyclic compounds of the present invention are helpful for recovery of the brain after injury.

Embodiment 71: Preventive and protective effects of knockout of TRPM2 channels on cerebral stroke

### 1) Preparation of the mouse transient focal cerebral ischemia middle cerebral artery embolism (tMCAO) model

After isoflurane inhalation gas anesthesia of 22-25 g male WT mice (TRPM2+/+) and TRPM2 knockout (TRPM2-/-) mice, the fiber-optic probes of laser Doppler flowmeter were fixed to the skull of the animals with glue and used to monitor the cerebral blood flow of the middle cerebral artery (MCA) supplying cortex.

The mice were fixed in the supine position, and the middle of the neck was dehaired and routinely sterilized with 75% alcohol. An approximately 1.5 cm incision was made in the middle of the neck of the mice, and the skin of the neck was incised, and the muscle and fascia were separated along the inner edge of the sternocleidomastoid muscle; The right carotid artery sheath was bluntly separated to expose the right common carotid artery (CCA), external carotid artery (ECA), and internal carotid artery (ICA); after the surgical scope was exposed satisfactorily, the CCA and ICA were temporarily clipped with arterial clips, and a small incision was made at the proximal end of the ECA with microsurgical scissors, and a nylon monofilament suture was inserted from the small incision along the ECA, and gently pushed into the intracranial area in the direction of the ICA for about 11.5 ± 0.5 mm, blocking the beginning of the middle cerebral artery. Only animals whose blood flow values decreased to 20% of the basic value were selected according to the blood flow data monitored by the flowmeter. After 90 minutes of ischemia, the monofilament sutures were gently withdrawn out, the arterial stumps were tied tightly, the surgical wounds were inspected, the bleeding was thoroughly stopped, a small amount of penicillin powder was sprinkled locally to prevent infection, and the subcutaneous tissues and the skin were sutured layer by layer, thus completing the ischemia reperfusion injury model. A heating plate was used to maintain the body temperature of the animals during surgery. After completion of the surgery, the sutured mice were placed in a heat preservation and humidification box until the mice recovered activity. Animals in the sham-operated group were subjected to the same surgical procedure but without middle artery occlusion.

### (3) Measurement of cerebral infarction volume

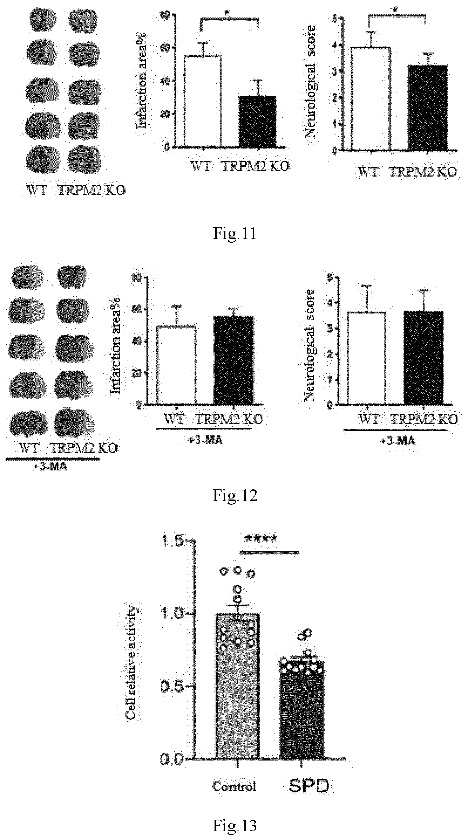

The mice were decapitated 24h after ischemia-reperfusion, the coronal planes of the brains were cut into brain slices of about 2mm thickness uniformly. The brain slices were placed in 0.25% 2,3,5-triphenyltetrazolium chloride (TTC) solution, incubated at 37°C Celsius for 30 minutes away from light, stained, and fixed in 4% paraformaldehyde neutral buffer (pH 7.4) overnight. The processed brain slices were photographed using a macro camera, and Image J image analysis software was used to calculate and count the cerebral infarct volume, and the measured results are shown in Fig. 11. The formula for calculating the percentage of infarct volume was (sum of the left uninfarcted area - sum of the right uninfarcted area)/sum of the left uninfarcted area×l00%.

(4) Transient middle cerebral artery occlusion model (tMCAO) was modeled with WT mice (TRPM2+/+) and TRPM2 knockout mice (TRPM2-/-), respectively. The results suggested that reduced TRPM2 levels could enhance autophagy during cerebral ischemia reperfusion and thus have a cerebroprotective effect, and this cerebroprotective effect could be reversed by the autophagy inhibitor 3-MA, and the results of the test are shown in Figure 12, suggesting that TRPM2 deficiency could have a protective effect by enhancing autophagy.

### Embodiment 72: Improvement of damage in the PD patient-derived iPSC differentiation dopaminergic neuron using TRPM2 Inhibitor II-23 and ACA(a TRPM2 non-specific inhibitor).

Using normal and disseminated PD patient-derived induced pluripotent stem cells (iPSC), performing drug interventions using the TRPM2 inhibitors ACA and II-23 after they were induced and differentiated into mature dopaminergic neurons, the drug treating method is as follows:
a. Calculating the materials for the experiment, and starting preparing when the dopaminergic neurons are differentiated to the 8th day. The petri dishes should be treated with 33 µg/mL PDL overnight and washed with sterilized water. After drying, 2 µg/mL of laminin was added and incubated overnight. Both PDL and laminin can be recycled for reuse.
b. Digesting and collecting the differentiated cells with Accutase, and counting with a cell counting plate, and then diluting to 500,000/mL with maturation medium (Y-27632 should be added). Fluorescence confocal imaging was performed using a confocal dish inoculated with 2 mL/dish, and cell viability assays were performed using a 96-well plate inoculated with 100 µL/well. Taking this time point as the 10th day, changing to Y-27632-free medium on the 11th day, and changing the medium every two days.
c. When the cells were cultured to the 15th day, treating the drugs to a final concentration with the medium, the working concentration of ACA is 0.5 µM, and the working concentration of II-23 is 0.6 µM, and the drug-containing medium was changed every four days.

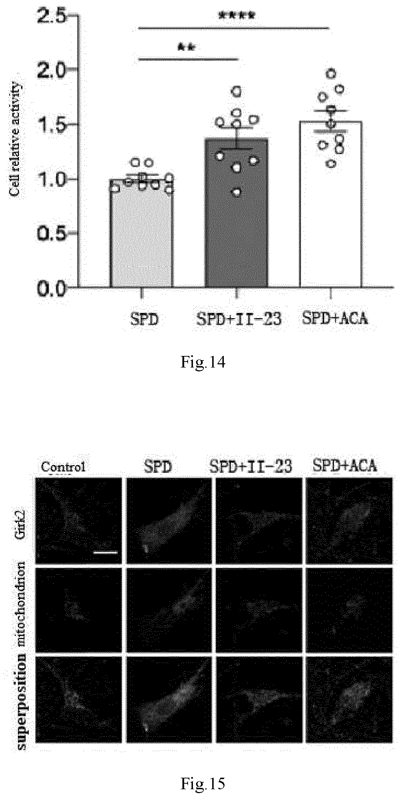

The experimental results were as follows:
(1) The viability of the differentiated dopaminergic neuronal cell is improved. Using cell titer glo (CTG kit, Promega), 100 µL/well of CTG reaction solution was added to a 96-well plate and incubated for 10 min at room temperature, and the fluorescence intensity was read and counted using an enzyme marker, the results of cell viability detection of the dopaminergic neurons derived from normal persons and disseminated PD patients after differentiation of induced pluripotent stem cells (iPSCs) are shown in Fig. 13, and the results of cell viability detection of the dopaminergic neurons derived from disseminated PD patients after differentiation of induced pluripotent stem cells (iPSC) treated with TRPM2 inhibitor II-23 and ACA are shown in Fig. 14.
   The results showed that the cell viability of the differentiated dopaminergic neurons derived from the disseminated PD patients was significantly decreased relative to the normal persons, and was restored after treatment with II-23 and ACA.
(2) the mitochondrial hyperfusion morphology of the differentiated DA neurons of derived from the PD patients are reduced.

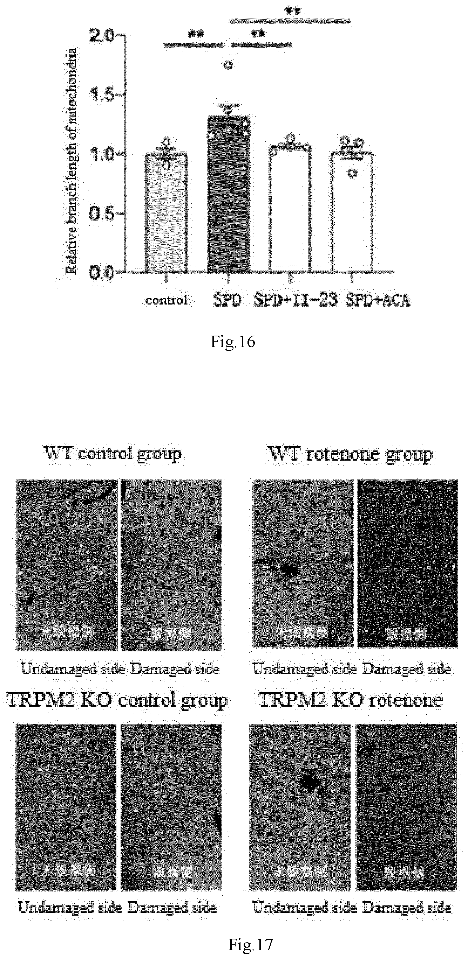

Positive dopamine neurons were labeled with a dopaminergic neuron marker molecule Girk2 antibody susceptible for SNc, and cellular mitochondria were labeled with the mitochondrial dye mitotracker as in Fig. 15. The specific method is as follow: dissolving Mito-tracker Red (25mM) in the mature DA neuron medium (1:500, working concentration of 50 uM), and adding the probe solution at a rate of each dish 700 µL and incubating at 37°C,30% CO2 for 90-120 min, then adding 700 ul of 4% PFA (1:1 with medium) and fixing for 10 min, then removing the PFA-medium mixture, adding 700 µL of 4% PFA and fixing for 10 min. Subsequently closing: PFA was suck away, washed twice with 1 ×PBS, and closed for 3 hours at room temperature with 1% BSA (with 0.1% Triton added) (preparation of closing solution: 0.5 g of BSA, fixing volum to 50 mL with PBS, filtering through 0.45 µm PVDF membrane, and then adding 50 µL of Triton, shaking well and then pre-cooling before use). After that, labeling with a primary antibody (dissolving the human-derived Girk2 primary antibody in the above closing solution (the ratio of 1:800-1:500 is recommended), adding the antibody at 400 ul per dish, and incubating at 4°C overnight) and labeling with a secondary antibody (removing the primary antibody, washing three times with 1×PBS, dissolving the secondary antibody in the above closing solution (the ratio of 1:1000 is recommended), adding the antibody at 400 µL per dish, and incubating at 4°C overnight),adding the antibody at 400 µL per dish and incubating at 4°C overnight. Finally, performing the fluorescence imaging: removing the secondary antibody, and washing three times with 1×PBS, adding 50% Glycerol for fluorescence protection, and then imaging with a Zeiss LSM880 laser confocal microscope, as shown in Fig. 15. The mitochondrial size in Fig. 15 was further statistically analyzed, and the results are shown in Fig. 16.

The results showed that dopaminergic neurons derived from the PD patients existed the excessive mitochondrial fusion, which could be reversed by using the TRPM2 inhibitors II-23 and ACA, thus indicating that the TRPM2 inhibitors were able to reduce the excessive mitochondrial fusion in the SPD control group.

### Embodiment 73: In vitro study of TRPM2 knockout on rotenone-induced injury and behavioral changes using TRPM2-KO mice (i.e., TRPM2 gene knockout mice).

### 1) Brain stereotactic injection

Anesthetizing C57-WT and TRPM-2-KO mice by intraperitoneal injection of 1% sodium pentobarbital (0.06 mL/10 g body weight) and fixing on a brain stereotaxic apparatus, and disinfecting the top of the head with iodophor and then cutting the surface hair of the head cleanly and disinfecting again with iodophor. Making a median sagittal incision, clipping the skins on both sides of the incision with arterial clips, and wiping with a cotton swab dipped in 30% H2O2, exposing the fontanel, determining the positon according to the mouse brain stereotaxic atlas (right medial forebrain bundle: AP -1.2mm ML 1.1mm DV 5.1mm, and right substantia nigra: AP -3.1mm ML 1.2mm DV 4.5mm ;) and punching with a micropore dental drill, slowly injecting with a micromolar injector (rotenone: 2.5 µg (2.5 µL, 1 µL/min); solvent: (2.5 µL, 1 µL/min)). At the end of the procedure, suturing and placing in a 37°C thermostatic incubator, and when they awoke, placing back in the cage and eating freely, and feeding sugar water or antibiotic dilution and adding wet food into the cage three days after the operation. Before surgery, injecting subcutaneously with 0.3 mL of saline to prevent intraoperative dehydration, applying erythromycin ophthalmic ointmen to eyes, measuring the anal temperature with a thermometer, and keeping warm with the insulated pads throughout the surgery.

### 2) Behavioral tests

### 2.1 Apomorphine-induced rotation behavioral test

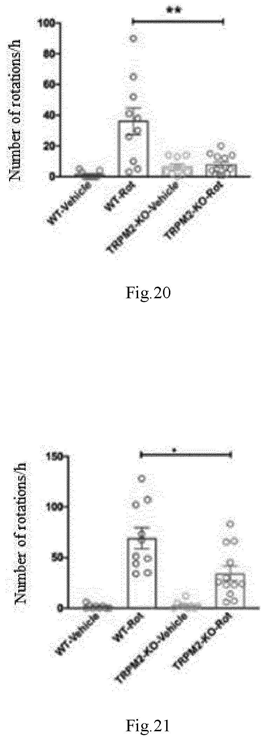

The apomorphine-induced rotation behavioral test was performed on the 7th and 14th day after rotenone modeling, respectively. First, the animals were placed in the test area to adapt to the surrounding environment for 10 minutes, after that, the rotation behavior was induced by subcutaneous injection of apomorphine solution (APO, 0.5 mg/kg) at the back of the neck. One 360° rotation was recorded as one cycle, and the number of cycles within 1 h was recorded. The environment was kept quiet throughout the test. The results of the apomorphine-induced rotation behavioral experiments are shown in Figures 20 and 21.

### 2.2 Rotating rod experiment

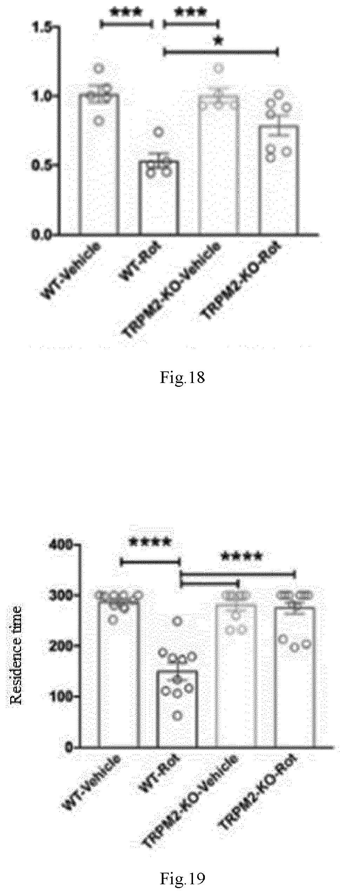

The rotating rod experiment behavioral test was used as an index of detecting motor coordination. The length of time that an animal stays on the rotating stick is an indicator of balance, coordination, body condition and locomotion. The longer the mice stayed on the rotating rod apparatus, the better their motor coordination. The statistical results of the rotating rod behavioral experiment are shown in Fig 18.

Acclimatization period: Placing the mice in the center of the rotating rod apparatus with their heads facing inward and their tails at a 45° angle, during the acclimatization period, training in the uniform speed mode, with the speed of the roller wheel at 5 rpm for 1 minute/times per mouse, three times a day.

Experimental period: After the mice were placed on the rotating rod apparatus to acclimatize for 2 minutes, starting the uniform acceleration experimental mode, with a rotational speed of 5~30 rpm for 5 minutes. When the experiment starts, the mice will continuously run on or fall down from the rotating rod apparatus. After 5 minutes, the rotating rod apparatus will stop automatically, and the system will record the time that the mice stayed on the rotating rod, and using discontinuous measurement methods, measuring the time again after an interval of two hours, taking the average of three measurements in total.

### 3) Immunohistochemistry

Mice were fixed after cardiac perfusion, and their brain tissues were removed and fixed in 4% PFA for 24h and then dehydrated in 30% sucrose for three days. Brain slices (20 µm/slice) were cut from the striatal region by a frozen slicing machine, and one slice was retained every six slices for immunohistochemistry staining of one index. After staining was completed, observing, quantifying, and photographing under a microscope, and the staining results obtained are shown in Fig. 17. The optical density values of the striatal TH staining were quantified using Image J image analysis software, and the results are shown in Fig. 18.

The specific steps of immunohisto fluorescence staining (floating method) were as follows: after the brain slices were washed by PBS (0.1 mol/L, pH 7.4), the sealed rupture membrane solution (4% BSA dissolved in 1% PBST) was placed in a horizontal shaking table at room temperature and processed for 2 hours. Without washing, the primary antibody was incubated overnight at 4°C in a chromatography cooler on a horizontal shaker (TH: 1:5000, stock number Sigma: MAB318). On the following day, all brain slices were rewarmed at room temperature for 2 hours, washed with PBS (5 min × 3), incubated with fluorescent mouse-derived secondary antibody for 2 hours away from light, washed with PBS (5 min × 3), picked up to the slides and then air-dried, and added the DAPI mounting medium containing about 60 µL for mounting.

### 4) Results

As shown in Figure 17 and Figure 18, tyrosine hydroxylase (TH) is the ratelimiting enzyme for dopamine synthesis, and the unilaterally disrupted substantia nigra and the disrupted intra-striatal TH-positive fibers of the medial forebrain of the mouse rotenone group (Rot) were significantly lower than those of the control group, whereas in the TRPM2-KO rotenone-modeled group, their intra-striatal TH-positive fibers were significantly increased compared with those in the WT rotenone modeled group, suggesting that TRPM2 gene knockout could protect the loss of intra-striatal TH-positive nerve fibers induced by rotenone, thereby ameliorating Parkinson's disease. As shown in Figure 19, the residence time on the rotating rod in the WT-ROT group was significantly lower compared to that in the WT control group, and the residence time on the rotating rod apparatus in the TRPM2-KO-ROT group was significantly higher compared to that in the WT-ROT group. As shown in Figures 20 and 21, the number of rotations per hour in the apomorphine-induced rotational behavioral experiments for the TRPM2-KO-ROT was significantly lower compared to that for the WT-ROT. Taken together, the above behavioral experiments indicate that TRPM2-KO protects against rotenone-induced behavioral changes.

According to the above experiments, it is shown that the fused ring heterocyclic compounds of the present invention, have an inhibitory effect on TRPM2 channels, and that the inhibition of TRPM2 channels can reduce the mitochondrial hyperfusion morphology of differentiated DA neurons derived from PD patients, and ameliorate the damage to the iPSC differentiated dopaminergic neurons derived from PD patient.

It can be understood by one of ordinary skill in the art that each of the above embodiments is a specific embodiment for realizing the present invention, and that various changes can be made thereto in form and detail in practical application without departing from the spirit and scope of the present invention.

## Claims

1. A fused heterocyclic compound, or a pharmaceutically acceptable salt, stereoisomer, solvates or prodrug thereof, wherein the fused heterocyclic compound has a structure shown in a general formula (I): 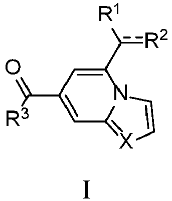
in the formula (I), R¹ is a heteroaryl group unsubstituted or with at least one hydrogen atom substituted by R¹⁻¹, or R¹ is a phenyl group unsubstituted or with at least one hydrogen atom substituted by R¹⁻², wherein R¹⁻¹ is a halogen or C₁₋₆ alkyl group, and R¹⁻² is a halogen, cyano, nitro group, C₂₋₈ ester group, C₁₋₆ alkyl group, C₁₋₆ alkoxy group;
represents a single or double bond, and when - - is a single bond, R² is a hydroxyl group, C₂₋₁₂ acyloxy group, or C₁₋₆ alkoxy group; When - - is a double bond, R² is an oxygen atom;
R³ is a hydroxyl group, C₁₋₄ alkyl group, C₁₋₆ alkoxy group, 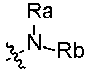 or a heterocyclic group containing at least one N, O and/or S atom, wherein Ra and Rb are independently selected from hydrogen, C₁₋₄ alkyl group, or C₁₋₄ alkoxy group, respectively;
X is a carbon atom or a nitrogen atom.

2. The fused heterocyclic compound according to claim 1, wherein the R¹ is a pentamembered heteroaryl group unsubstituted or with at least one hydrogen atom substituted by R¹⁻¹, or the R¹ is a hexamembered heteroaryl group unsubstituted or with at least one hydrogen atom substituted by R¹⁻¹, wherein the R¹⁻¹ is a halogen or C₁₋₄ alkyl group;
or, the R¹ is a phenyl group unsubstituted or with at least one hydrogen atom substituted by R¹⁻², wherein the R¹⁻² is a halogen, cyano, nitro, C₁₋₄ alkyl, C₁₋₄ alkoxy group;
and/or, represents a single bond, the R² is a hydroxyl group, C₂₋₈ acyloxy group, or C₁₋₄ alkoxy group;
or, represents a double bond, the R² is an oxygen atom;
and/or, R³ is a hydroxyl group, C₁₋₄ alkyl group, C₁₋₄ alkoxy group, a ternary heterocyclic group containing at least one N, O and/or S atom, a quaternary heterocyclic group containing at least one N, O and/or S atom, a pentamembered heterocyclic group containing at least one N, O and/or S atom, a hexamembered heterocyclic group containing at least one N, O and/or S atom or 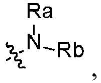 wherein Ra and Rb are independently selected from hydrogen, methyl group, ethyl group, methoxy group or ethoxy group, respectively.

3. The fused heterocyclic compound according to claim 1, wherein the R¹ is a pentamembered heteroaryl group unsubstituted or with at least one hydrogen atom substituted by R¹⁻¹, or the R¹ is a pyridyl group unsubstituted or with at least one hydrogen atom substituted by R¹⁻¹, wherein the R¹⁻¹ is fluorine, chlorine, bromine, iodine, methyl, ethyl, n-propyl, isopropyl, n-butyl, isobutyl or tert butyl;
or, the R¹ is a phenyl group unsubstituted or with at least one hydrogen atom substituted by R¹⁻², wherein R¹⁻² is fluorine, chlorine, bromine, iodine, methyl, ethyl, n-propyl, isopropyl, n-butyl, isobutyl, tert butyl, methoxy or ethoxy,
and/or, represents a single bond, the R² is a hydroxyl group, methoxy group, ethoxy group, propoxy group or 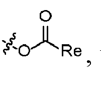 where Re is a C₁₋₇ alkyl group;
or, represents a double bond, R² is an oxygen atom;
and/or, R³ is a hydroxyl group, methyl group, ethyl group, n-propyl group, isopropyl group, n-butyl group, isobutyl or tert butyl group, C1-4 alkoxy group, a quaternary heterocyclic group containing at least one N and/or O atom, a pentamembered heterocyclic group containing at least one N and/or O atom, and a hexamembered heterocyclic group containing at least one N and/or O atom,

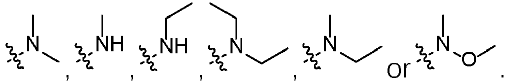

4. The fused heterocyclic compound according to claim 1, wherein the fused heterocyclic compound has a structure shown in a general formula (II): 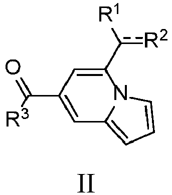
in the formula (II), R¹ is a heteroaryl group unsubstituted or with at least one hydrogen atom substituted by R¹⁻¹,or R¹ is aphenyl group unsubstituted or with at least one hydrogen atom substituted by R¹⁻², wherein R¹⁻¹ is a halogen or C₁₋₆ alkyl group, and R¹⁻² is a halogen, cyano, nitro group, C₁₋₆ alkyl group, C₁₋₆ alkoxy group;
represents a single or double bond, and when - - is a single bond, R² is a hydroxyl group, C₂₋₁₂ acyloxy group, or C₁₋₆ alkoxy group; When - - is a double bond, R² is an oxygen atom;
R³ is a hydroxyl group, C₁₋₄ alkyl group, C₁₋₆ alkoxy group, 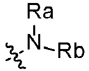 or a heterocyclic group containing at least one N, O and/or S atom, wherein Ra and Rb are independently selected from hydrogen, C₁₋₄ alkyl group or C₁₋₄ alkoxy group, respectively.

5. The fused heterocyclic compound according to claim 1, wherein the fused heterocyclic compound has a structure shown in a general formula (III): 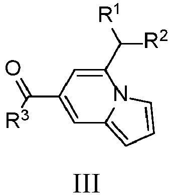
in the formula (III), R¹ is a phenyl group unsubstituted or with at least one hydrogen atom substituted by R¹⁻², wherein R¹⁻² is a halogen, cyano, nitro group, C₁₋₆ alkyl group, C₁₋₆ alkoxy group;
R² is a hydroxyl group, C₂₋₁₂ acyloxy group, or C₁₋₆ alkoxy group;
R³ is a hydroxyl group, C₁₋₄ alkyl group, C₁₋₆ alkoxy group, 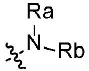 ,wherein Ra and Rb are independently selected from hydrogen, C₁₋₄ alkyl group, or C₁₋₄ alkoxy group, respectively.

6. The fused heterocyclic compound according to claim 4, wherein the fused heterocyclic compound has a structure shown in a general formula (IV): 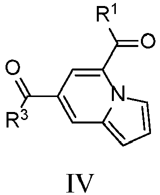
in the formula (IV), R¹ is a heteroaryl group unsubstituted or with at least one hydrogen atom substituted by R¹⁻¹, or R¹ is a phenyl group unsubstituted or substituted by R¹⁻², wherein R¹⁻¹ is a halogen or C₁₋₆ alkyl group, and R¹⁻² is a halogen, cyano, nitro group, C₁₋₆ alkyl group, C₁₋₆ alkoxy group;
R³ is a hydroxyl group, C₁₋₄ alkyl group, C₁₋₆ alkoxy group, 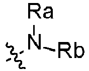 or a heterocyclic group containing at least one N, O and/or S atom, wherein Ra and Rb are independently selected from hydrogen, C₁₋₄ alkyl group, or C₁₋₄ alkoxy group, respectively.

7. The fused heterocyclic compound according to claim 4, wherein the fused heterocyclic compound has a structure shown in a general formula (IV): 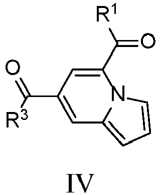
in the formula (IV), R¹ is a pentamembered heterocyclic group unsubstituted, a hexamembered heterocyclic group unsubstituted, or a phenyl group unsubstituted or with at least one hydrogen atom substituted by R¹⁻², wherein R¹⁻² is a halogen, C₁₋₆ alkyl group, C₁₋₆ alkoxy group;
R³ is a C₁₋₄ alkyl group, C₁₋₆ alkoxy group.

8. The fused heterocyclic compound according to any one of claims 1 to 4, wherein the fused heterocyclic compound is selected from any of the following structures:

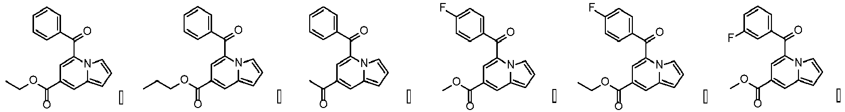

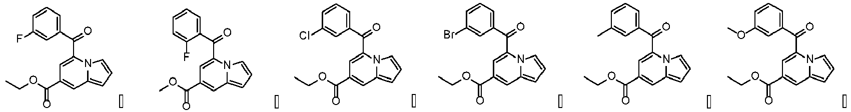

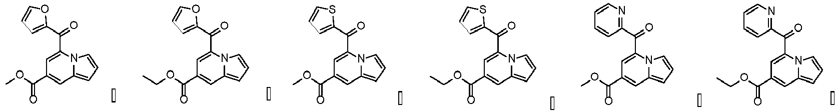

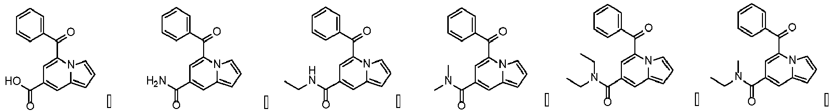

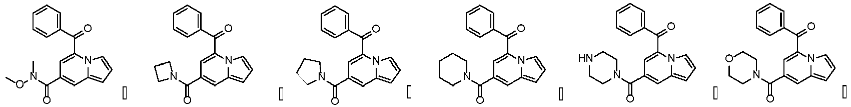

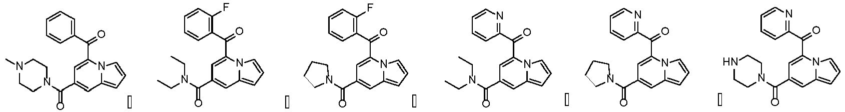

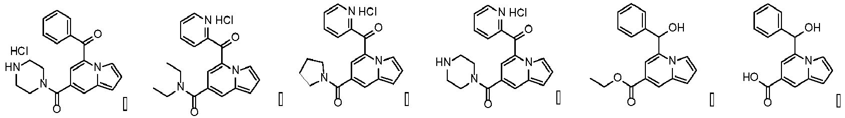

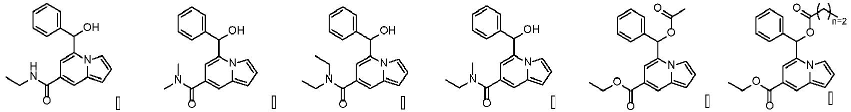

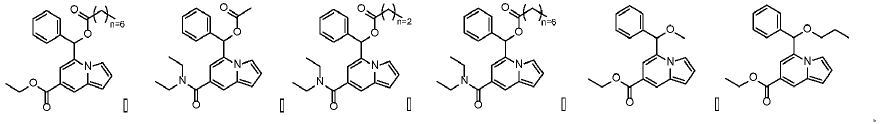

9. The fused heterocyclic compound according to claim 1, wherein X is an N atom in the structure of fused heterocyclic compounds.

10. The fused heterocyclic compound according to claim 9, wherein the fused heterocyclic compound has a structure shown in a general formula (V): 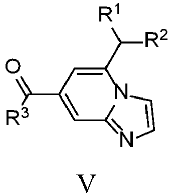
in the formula(V), R¹ is a phenyl group unsubstituted or with at least one hydrogen atom substituted by R¹⁻², wherein R¹⁻² is a halogen, cyano, nitro group, C₁₋₆ alkyl group , C₁₋₆ alkoxy group;
R³ is a hydroxyl group, C₁₋₄ alkyl group or C₁₋₆ alkoxy group.

11. The fused heterocyclic compound according to claim 9, wherein the fused heterocyclic compound has a structure shown in a general formula (VI): 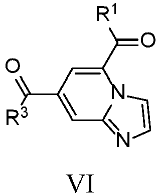
in the formula (VI), R¹ is a phenyl group unsubstituted or with at least one hydrogen atom substituted by R¹⁻², wherein R¹⁻² is a halogen, cyano, nitro group, C₁₋₆ alkyl or C₁₋₆ alkoxy group;
R³ is a hydroxyl group, C₁₋₄ alkyl group, C₁₋₆ alkoxy group, a heterocyclic group containing at least one N atom, or 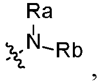 wherein Ra and Rb are independently selected from hydrogen or C₁₋₄ alkyl group, respectively.

12. The fused heterocyclic compound according to claim 9, wherein the fused heterocyclic compound has the structure shown in general formula (VI): 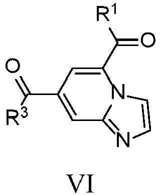
in the formula (VI), R¹ is a phenyl group unsubstituted or substituted by halogen;
R³ is a hydroxyl group, C₁₋₄ alkyl group, C₁₋₄ alkoxy group.

13. The fused heterocyclic compound according to any one of claims 1 to 3 and 9 to 11, wherein the fused heterocyclic compound is selected from any of the following structures:

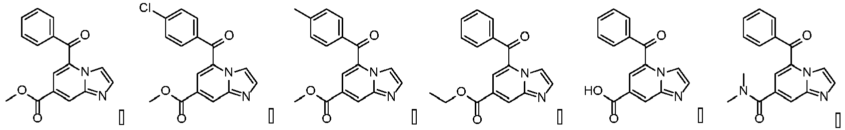

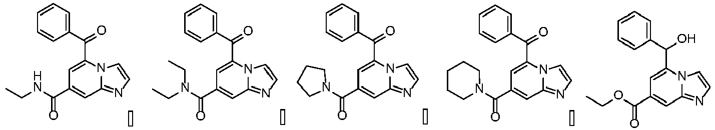

14. A pharmaceutical composition, wherein the pharmaceutical composition comprises the fused heterocyclic compound according to any one of claims 1-13, or a pharmaceutically acceptable salt, stereoisomer, solvate or prodrug thereof.

15. The application of a fused heterocyclic compound according to any one of claims 1-13, or a pharmaceutically acceptable salt, stereoisomer, solvate or prodrug thereof in the preparation of TRPM2 inhibitor drugs.

16. The application according to claim 15, wherein the TRPM2 dysfunctional diseases include:
spinal cord injury or pain, cardiovascular and cerebrovascular diseases, Alzheimer's disease, neuropathic pain, Parkinson's disease, bipolar disorder or amyotrophic lateral sclerosis, liver and kidney ischemic diseases.

17. The application according to claim 16, wherein the cardiovascular and cerebrovascular diseases are caused by oxidative stress caused by ischemia/reperfusion.

18. The application according to claim 16 or 17, wherein the cardiovascular and cerebrovascular diseases include apoplexy, cerebral thrombosis, cerebral stroke, coronary heart disease, myocardial infarction, or heart failure.

19. Application of the pharmaceutical composition according to claim 14 in the preparation of TRPM2 inhibitor drugs.

20. A method for preparing a fused heterocyclic compound according to claim 7, wherein the method comprises step (1):
under alkaline conditions, M1 and 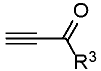 carry out the following reaction to obtain the fused heterocyclic compound; 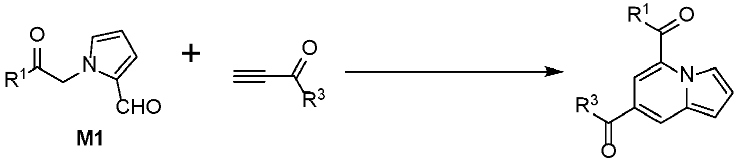
wherein R¹ is a pentamembered heterocyclic group unsubstituted, a hexamembered heterocyclic group unsubstituted, a phenyl group unsubstituted or with at least one hydrogen atom substituted by R¹⁻², wherein R¹⁻² is a halogen, C₁₋₆ alkyl group or C₁₋₆ alkoxy group;
R³ is a C₁₋₄ alkyl group or C₁₋₆ alkoxy group.

21. A method for preparing a fused heterocyclic compound according to claim 12, wherein the method comprises step (a) :
under alkaline conditions, M2 and 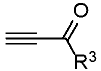 carry out the following 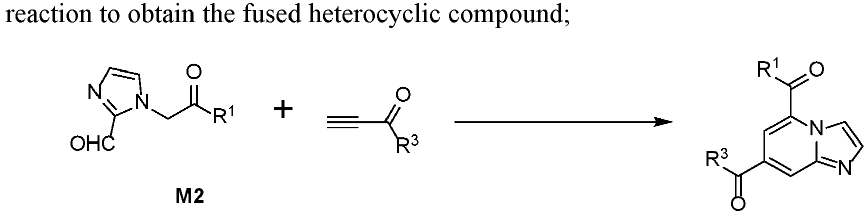
wherein R¹ is a phenyl group unsubstituted or substituted by halogen;
R³ is a hydroxyl group, C₁₋₄ alkyl group or C₁₋₄ alkoxy group.

22. The fused heterocyclic compound according to claim 1, wherein the fused heterocyclic compound is not any of the following structures:
